# EUROPEAN PATENT APPLICATION

(11) **EP 4 541 315 A1**
(43) Date of publication of application: **23.04.2025**
(21) Application number: 23838757.5
(22) Date of filing: 30.06.2023
(51) Int. Cl.: A61F 2/24

(54) **HEART VALVE STENT AND PROSTHETIC HEART VALVE**

(30) Priority: 12.07.2022 CN 202210820049; 12.07.2022 CN 202210820044; 12.07.2022 CN 202210822410; 03.03.2023 CN 202310203899
(71) Applicant: Mitrassist Lifesciences Limited, Shanghai 201807 (CN)
(72) Inventor: LIANG, Xiangbin, Shanghai 201807 (CN); ZHONG, Wei, Shanghai 201807 (CN); WANG, Song, Shanghai 201807 (CN)
(74) Representative: Berggren Oy
(86) International application number: PCT/CN2023/104741
(87) International publication number: WO 2024/012265

(57) **Abstract**

The present invention relates to the technical field of medical devices, and provides an artificial/implantable heart valve stent and a prosthetic heart valve. The heart valve stent comprises supporting units and an extension branch; the supporting units define a flow channel for blood to flow therethrough; the extension branch extends from the supporting units to the outside of the flow channel, and is provided with an extension part abutting against the heart tissue; a gap for accommodating a native heart valve leaflet is formed between the extension branch and a supporting main body. According to the technical solution in the present invention, the stability and reliability of installation and fixation of the heart valve stent can be improved, and the service life of the heart valve stent is prolonged, thereby reducing patients' risk of replacing the valve again; moreover, it is conducive to reducing coronary artery blockage when patients use heart valve stents.

## Description

### CROSS-REFERENCE TO RELATED APPLICATION

The present invention claims priorities to Chinese Patent Application No. CN202210820044.3, titled "IMPLANTABLE HEART VALVE STENT AND PROSTHETIC HEART VALVE," filed on July 12, 2022 with the Chinese Patent Office, Chinese Patent Application No. CN202210820049.6, titled "HEART VALVE STENT AND PROSTHETIC HEART VALVE," filed on July 12, 2022 with the Chinese Patent Office, Chinese Patent Application No. CN202210822410.9, titled "ARTIFICIAL HEART VALVE STENT AND PROSTHETIC HEART VALVE," filed on July 12, 2022 with the Chinese Patent Office, and Chinese Patent Application No. CN2023102038996, titled "HEART VALVE STENT AND PROSTHETIC HEART VALVE," filed on March 03, 2023 with the Chinese Patent Office, the entire contents of which are incorporated by reference into the present invention.

### TECHNICAL FIELD

The present invention relates to the technical field of medical devices, and specifically to a heart valve stent and a heart valve prosthesis (i.e., a prosthetic heart valve).

### BACKGROUND ART

The heart valves grow between the atria and the ventricles, and between the ventricles and the aorta, and play a role of one-way valve for helping the unidirectional movement of the blood flow. The four heart valves in the body are called as the mitral valve, tricuspid valve, aortic valve, and pulmonary valve respectively. If these heart valves are diseased (e.g., become narrowed or closed insufficiently), the movement of blood flow will be affected, which will result in the abnormal function of the heart, and finally result in heart failure.

At present, when the heart valve is diseased, it is mostly treated by using the valve replacement surgery, i.e., it is replaced by the artificial mechanical valve or the biological valve. However, the existing heart valve stent after implanted is easy to fall off under the influence of the blood flow, so that the stability of the installation and fixation is poor, which affects the service life of artificial heart valve, so as to increase the risk of the replacement of the valve again for the patient; and the phenomenon of coronary artery blockage is easily occurred after the installation of partial heart valve stents.

### SUMMARY

The present invention provides a heart valve stent, including a supporting body and at least one protrusion branch body connected to the supporting body, wherein a flow channel for blood flow is defined by the supporting body; and the protrusion branch body extends from the supporting body to an outer side of the flow channel to form a protrusion part that can abut against the heart tissue, and a gap that can accommodate a native heart valve leaflet is formed between the protrusion branch body and the supporting body.

Optionally, the supporting body includes a plurality of supporting units, and the flow channel is formed by the plurality of supporting units, wherein
each supporting unit includes two first supporting bodies for connecting different valve leaflets respectively and a second supporting body connecting the two first supporting bodies, and a space that can be connected and covered by a skirt is formed between the two first supporting bodies and the second supporting body.

The present invention further provides a heart valve stent, wherein the heart valve stent is woven and shaped by at least one elongated material, and a flow channel for blood flow is defined by the heart valve stent, wherein the at least one elongated material protrudes towards an outer side of the flow channel to form a protrusion part that can abut against the heart tissue.

Optionally, the heart valve stent includes a plurality of supporting units, and the flow channel is formed by the plurality of supporting units, wherein
each supporting unit includes two first supporting bodies for connecting different valve leaflets respectively and a second supporting body connecting the two first supporting bodies, and a space that can be connected and covered by a skirt is formed between the two first supporting bodies and the second supporting body.

Optionally, upstream and downstream directions are defined according to a direction of blood passing through the flow channel, wherein the second supporting body is located in the upstream direction of the two first supporting bodies.

Optionally, the two first supporting bodies extend in the downstream direction from two ends of the second supporting body respectively, and the two first supporting bodies are converged and connected.

Optionally, the two first supporting bodies extend in the downstream direction from each end of the second supporting body respectively and second ends of the two first supporting bodies are connected.

Optionally, each supporting unit is connected to the protrusion branch body, and the protrusion branch body is located between two adjacent supporting units in a circumferential direction of the supporting body.

Optionally, each supporting unit is connected to two protrusion branch bodies, and two protrusion branch bodies between two adjacent supporting units are connected to each other.

Optionally, two protrusion branch bodies between two adjacent supporting units are formed by one braided wire.

Optionally, a connecting ring is formed in the downstream direction of the first supporting body.

Optionally, the connecting ring is formed on each first supporting body.

Optionally, a first riveted knot is arranged on an upstream part of the connecting ring, and the connecting ring forms a closed ring by the first riveted knot.

Optionally, each supporting unit is connected to an adjacent supporting unit by a riveted structure to form a second riveted knot, wherein in the second riveted knot, a first supporting body and a second supporting body of two adjacent supporting units are arrayed in parallel, and the gap accommodating the native heart valve leaflet is formed between the protrusion branch body and the first supporting body and the second supporting body located in the second riveted knot.

Optionally, the two first supporting bodies of each supporting unit are connected by the riveted structure to form a third riveted knot.

Optionally, the protrusion branch body includes continuous protrusion section and connection section in a direction that the blood passes through the flow channel,

wherein the protrusion section bends and extends in a direction away from the flow channel; and one end of the connection section is connected to the protrusion section; the other end is connected to the supporting body; and an angle between the protrusion section and the flow channel in an axial direction is in a range of 1°~150°.

Optionally, a horizontal distance a between one end of the connection section connected to the protrusion section and the supporting body located upstream the flow channel is set in a range of 1 mm~20 mm.

Optionally, the heart valve stent is woven by at least one elongated material.

Optionally, each supporting unit further includes a third supporting body, and a connecting ring located downstream the heart valve stent is formed by the third supporting body.

Optionally, one end of the third supporting body is connected to the first supporting body, and the other end forms the protrusion part.

Optionally, each supporting unit includes two third supporting bodies, wherein one end of each of the two third supporting bodies is connected to one of the two first supporting bodies respectively, and the other end of each of the two third supporting bodies forms the protrusion part.

Optionally, one connecting ring is formed at the most downstream part of each third supporting body.

Optionally, a protrusion part of each supporting unit and a protrusion part of the adjacent supporting unit are formed by bending the same elongated material, and two adjacent protrusion parts are continuous.

Optionally, each supporting unit is connected to the adjacent supporting unit by a riveted structure to form a first riveted knot, wherein in the first riveted knot, individual elongated materials are arrayed in parallel, and the two protrusion parts are located at the middle.

Optionally, two first supporting bodies of each supporting unit are connected by a riveted structure to form a second riveted knot, and the third supporting body is also connected to the second riveted knot.

Optionally, an angle between the protrusion direction of the protrusion part and a direction perpendicular to the axial direction of the flow channel is in a range of 15°~90°.

Optionally, in the direction that the blood passes through the flow channel, the third supporting body includes continuous protrusion section, transition section, and connection section, wherein
the protrusion part is formed by the protrusion section; one end of the transition section is connected to the protrusion section; the other end bends and extends in a direction away from the flow channel, and has an angle in a range of 60°~150° with a direction perpendicular to the axial direction of the flow channel; and one end of the connection section is connected to the transition section, and the other end is connected to the first supporting body.

Optionally, the elongated material includes a memory alloy wire.

Optionally, the second supporting body is woven by a variable-diameter memory alloy wire; or
a memory alloy tube is embedded at a part of an outer peripheral side of the second supporting body.

The present invention further provides an implantable heart valve stent, wherein
the implantable heart valve stent includes a plurality of supporting units, and a flow channel for blood flow is formed by the plurality of supporting units, wherein
at least one of the supporting units includes a protrusion branch body; a protrusion part that can abut against the heart tissue is formed by the protrusion branch body protruding in a direction away from the flow channel; and the protrusion branch body extends in an upstream direction of the flow channel to form a connecting body that can connect to a skirt.

Optionally, each supporting unit includes two first supporting bodies respectively connecting different valve leaflets, and a first space that can be connected and covered by the skirt is formed between two first supporting bodies and the connecting body.

Optionally, upstream and downstream directions are defined according to a direction of blood passing through the flow channel, wherein the connecting body is located at the upstream direction of the two first supporting bodies.

Optionally, the connecting body includes at least one sub-connecting body, and the at least one sub-connecting body and the connecting body are stacked or arranged at intervals.

Optionally, the two first supporting bodies extend in the downstream direction from two ends of the connecting body respectively, and the two first supporting bodies are converged and connected.

Optionally, each supporting unit includes the protrusion branch body, and the protrusion branch body is located between two adjacent supporting units in a circumferential direction of the flow channel.

Optionally, each supporting unit includes two protrusion branch bodies, and connecting bodies formed by the two protrusion branch bodies are connected to each other.

Optionally, a second space for a medical device to pass through is formed between each protrusion branch body and the first supporting body.

Optionally, the protrusion branch body and the first supporting body are woven by one braided wire.

Optionally, a connecting ring is formed at one end of the protrusion branch body located downstream the flow channel.

Optionally, each supporting unit is connected to the adjacent supporting unit by the riveted structure to form the first riveted knot, wherein in the first riveted knot, a first supporting body of two adjacent supporting units and a connecting body are arrayed in parallel.

Optionally, two first supporting bodies of each supporting unit are connected by the riveted structure to form a second riveted knot.

Optionally, the protrusion branch body includes a first connection section, an abutting section, and a second connection section connected sequentially, and the first connection section is connected to the first riveted knot, wherein a first end of the abutting section is connected to the first connection section, and a second end protrudes in a direction away from the flow channel; and one end of the second connection section is connected to the second end of the abutting section, and the other end is connected to the first supporting body.

Optionally, an angle α between the abutting section and the axis direction of the flow channel is in a range of 10°~150°.

Optionally, a distance b between the second end of the abutting section and the first connection section is in a range of 1 mm~20 mm.

Optionally, the implantable heart valve stent is woven by at least one braided wire.

Optionally, the braided wire includes a memory alloy wire.

Optionally, the connecting body is woven by a variable-diameter memory alloy wire; or
a memory alloy tube is embedded at a part of an outer peripheral side of the connecting body.

The present invention further provides a heart valve prosthesis, including the heart valve stent in any one of the above or the implantable heart valve stent in any one of the above, including:
a valve leaflet, arranged in the flow channel and connected to the first supporting body of the heart valve stent or the implantable heart valve stent; and
a first sealing skirt, connecting and covering the space formed between two first supporting bodies and the second supporting body of the heart valve stent or the implantable heart valve stent.

Optionally, the second sealing skirt is formed around the outer peripheral side of the heart valve stent or the implantable heart valve stent.

Optionally, the second sealing skirt is in a shape of a disc, and a flange is formed by folding a peripheral side of the second sealing skirt towards downstream the heart valve stent.

Optionally, the valve leaflet is made of a material including at least one of a polymer material, a biological tissue material, and a tissue engineering material.

Optionally, a connection method of the valve leaflet and the first supporting body of the heart valve stent or the implantable heart valve stent is one of suture stitching, bonding, heat fusion, and polymer attachment.

The present invention further provides a heart valve stent, including:
a supporting body, including a plurality of supporting units, wherein a channel for blood flow is formed by the plurality of supporting units; each supporting unit includes two first supporting bodies respectively connecting different valve leaflets; at least one of the supporting units includes a second supporting body; the second supporting body is arranged on one side of the first supporting body close to an adjacent supporting unit; and
at least one protrusion branch body, wherein the protrusion branch body is fixed relative to two adjacent supporting units and extends from the supporting body to the outer side of the channel, and a gap for accommodating the native heart valve leaflet is formed between the protrusion branch body and the supporting body.

Optionally, each supporting unit includes two second supporting bodies, and two second supporting bodies in each supporting unit are arranged on two sides of one of the two first supporting bodies respectively.

Optionally, each supporting unit includes a third supporting body connecting two first supporting bodies, and a space that can be connected and covered by the first skirt is formed between the two first supporting bodies and the third supporting body.

Optionally, each first supporting body is fixed relative to one protrusion branch body; and each protrusion branch body is fixedly connected relatively to two adjacent first supporting bodies belonging to two adjacent supporting units respectively; and a protrusion part for abutting against the heart tissue is formed at a middle part of the protrusion branch body.

Optionally, two adjacent second supporting bodies respectively belonging to two adjacent supporting units are located between protrusion branch bodies connecting the two supporting units in a circumferential direction of the heart valve stent.

Optionally, two adjacent second supporting bodies respectively belonging to two adjacent supporting units are located at an inner side of the protrusion branch bodies connecting the two supporting units in a radial direction of the heart valve stent.

Optionally, a downstream part of two first supporting bodies of the supporting unit and a downstream part of two protrusion branch bodies are connected by the riveted structure, so as to form the first riveted knot, wherein two protrusion branch bodies are two protrusion branch bodies fixed relative to the two first supporting bodies.

Optionally, each second supporting body is fixed relative to one protrusion branch body, and the protrusion part for abutting against the heart tissue is formed at the middle part of the protrusion branch body.

Optionally, parts of two adjacent second supporting bodies respectively belonging to two adjacent supporting units are located between protrusion branch bodies connecting the two supporting units in a circumferential direction of the heart valve stent.

Optionally, parts of two adjacent second supporting bodies respectively belonging to two adjacent supporting units are located at an inner side of the protrusion branch bodies connecting the two supporting units in a radial direction of the heart valve stent.

Optionally, downstream parts of two first supporting bodies of the supporting unit are connected by the riveted structure to form the first riveted knot; and a downstream part of the protrusion branch body and the second supporting body are connected by the riveted structure to form the second riveted knot.

Optionally, upstream parts of two adjacent first supporting bodies, upstream parts of two adjacent second supporting bodies, and downstream parts of two adjacent third supporting bodies respectively belonging to two adjacent supporting units are connected by riveted structures, so as to form the third riveted knot.

Optionally, each supporting unit is woven by one braided wire, and each protrusion branch body is formed by another braided wire.

Optionally, the braided wire includes a variable-diameter part, wherein a diameter of the variable-diameter part is larger than that of the other parts, and the variable-diameter part is arranged corresponding to the riveted structure.

Optionally, the braided wire is made of the variable-diameter memory alloy wire to form the variable-diameter part; or
a memory alloy tube is embedded at an outer periphery of the braided wire, wherein a part having the memory alloy tube is the variable-diameter part.

Optionally, a connecting ring is formed in a downstream direction of each supporting unit.

The present invention further provides a heart valve prosthesis, including the heart valve stent according to any one of the above, including:
a valve leaflet, arranged in the channel and connected to the first supporting body of the heart valve stent; and
a first skirt, connecting and covering the space formed between two first supporting bodies and the third supporting body of the supporting unit of the heart valve stent.

Optionally, the heart valve prosthesis further includes a second skirt, wherein the second skirt is arranged around the outer peripheral side of the heart valve stent; an upstream end of the second skirt is connected to the first skirt; and a circumference of the second skirt from an upstream end to a downstream end gradually becomes larger and then gradually becomes smaller.

Optionally, an annular first flange is formed on a downstream end of the second skirt, and the first flange faces downstream the heart valve stent.

Optionally, an accommodation notch is formed on the first flange and the second skirt, and the accommodation notch is configured to accommodate the supporting body.

Optionally, an annular second flange is formed at a peripheral side of the second skirt, and the second flange faces downstream the heart valve stent.

Optionally, the second flange is arranged on a peripheral side of a largest circumference of the second skirt.

### BRIEF DESCRIPTION OF DRAWINGS

In order to more clearly illustrate the technical solutions in the embodiments of the present invention, the drawings to be used in the embodiments will be briefly introduced below. It should be understood that the following drawings only show certain embodiments of the present invention, and therefore should not be regarded as a limitation of the scope. For a person of ordinary skill in the art, other relevant drawings can be obtained according to these drawings without inventive efforts.
FIG. 1 shows a structure schematic diagram of a heart valve stent at one view provided by some embodiments of the present invention;
FIG. 2 shows a structure schematic diagram of a heart valve stent at the other view provided by some embodiments of the present invention;
FIG. 3 shows an enlarged schematic structure schematic diagram of part A in FIG. 2;
FIG. 4 is a partial schematic diagram of a second supporting body provided by an embodiment of the present invention;
FIG. 5 is a partial schematic diagram of a second supporting body provided by another embodiment of the present invention;
FIG. 6 shows a structure schematic diagram of a heart valve prosthesis provided by some embodiments of the present invention;
FIG. 7 shows a structure schematic diagram of a heart valve stent at one view provided by another embodiment of the present invention;
FIG. 8 shows a structure schematic diagram of a heart valve stent at the other view provided by another embodiment of the present invention;
FIG. 9 shows an enlarged structure schematic diagram of part A in FIG. 8;
FIG. 10 shows a structure schematic diagram of a heart valve prosthesis provided by another embodiment of the present invention;
FIG. 11 shows a structure schematic diagram of an implantable heart valve stent at one view provided by embodiments of the present invention;
FIG. 12 shows a structure schematic diagram of an implantable heart valve stent at the other view provided by embodiments of the present invention;
FIG. 13 shows an enlarged structure schematic diagram of part A in FIG. 12;
FIG. 14 shows a connection structure schematic diagram of a connecting body provided by embodiments of the present invention;
FIG. 15 shows the other connection structure schematic diagram of a connecting body provided by embodiments of the present invention;
FIG. 16 shows a structure schematic diagram of a heart valve prosthesis provided by another embodiment of the present invention;
FIG. 17 shows a schematic diagram of a three-dimensional structure of a heart valve stent provided by another embodiment of the present invention;
FIG. 18 shows a schematic diagram of a three-dimensional structure of a heart valve stent at the other view provided by another embodiment of the present invention;
FIG. 19 shows a schematic diagram of a three-dimensional structure of a heart valve stent, a valve leaflet, and a first skirt provided by embodiments of the present invention;
FIG. 20 shows a schematic structure of a braided wire of a heart valve stent provided by embodiments of the present invention;
FIG. 21 shows a schematic structure of a braided wire of another heart valve stent provided by embodiments of the present invention;
FIG. 22 shows a schematic diagram of a three-dimensional structure of another heart valve stent provided by embodiments of the present invention;
FIG. 23 shows a schematic diagram of a three-dimensional structure of another heart valve stent at the other view provided by embodiments of the present invention;
FIG. 24 shows a structure schematic diagram of a heart valve prosthesis provided by the embodiments of the present invention;
FIG. 25 shows a schematic diagram of a three-dimensional structure of a second skirt in a heart valve prosthesis provided by embodiments of the present invention; and
FIG. 26 shows a schematic diagram of a three-dimensional structure of a second skirt in the other heart valve prosthesis provided by embodiments of the present invention.

Reference numbers:
heart valve prosthesis 1; heart valve stent or (artificial) heart valve stent or implantable heart valve stent 10; supporting body 100; supporting unit 110; first supporting body 111; second supporting body 112; third supporting body 113; first riveted knot 114; second riveted knot 115; third riveted knot 116; flow channel or channel 120; braided wire 131; variable-diameter part 132; braided wire 133; variable-diameter part 134; protrusion branch body 200; protrusion part 210; protrusion section 220; connection section 230; transition section 240; connecting body 250; sub-connecting body 251; gap 300; connecting ring 400; memory alloy tube 500; first space 600-A; second space 600-B; valve leaflet 20; first sealing skirt 30; second sealing skirt 40;
supporting body 800; supporting unit 810; first supporting body 811; second supporting body 812; third supporting body 813; first riveted knot 814; second riveted knot 815; third riveted knot 816; protrusion branch body 900; protrusion part 910; valve leaflet 20; first skirt 50; second skirt 60; first flange 601; accommodation notch 602; second skirt 70; first flange 701; accommodation notch 702; and second flange 703.

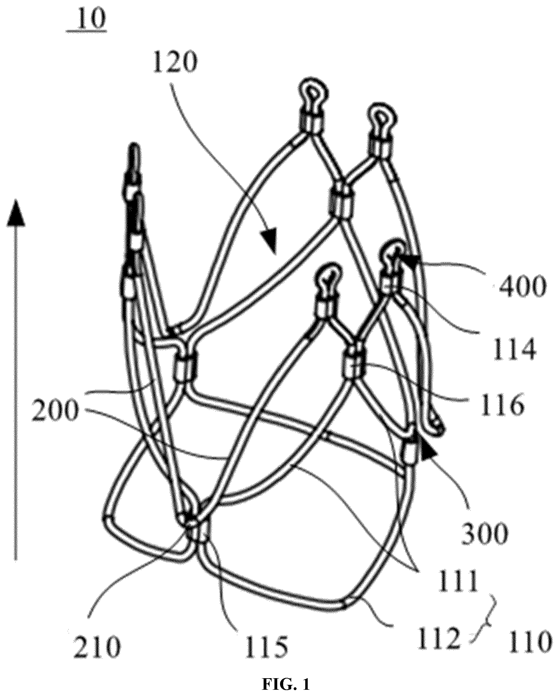

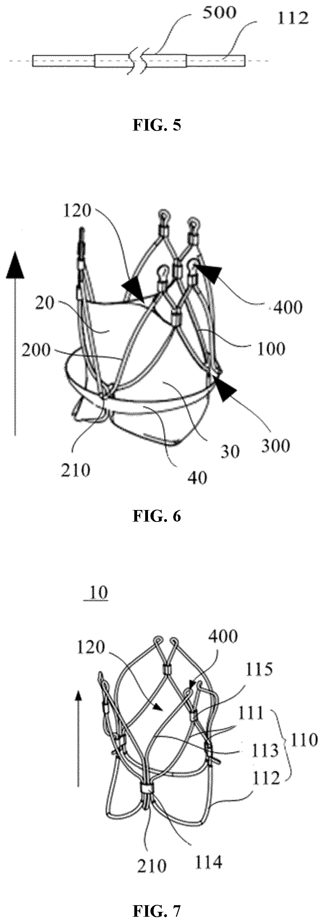

The direction of the arrow in FIG. 1 and FIG. 6 shows the direction of the blood flow.

### DETAILED DESCRIPTION OF EMBODIMENTS

The technical solutions in the embodiments of the present invention will be clearly and completely described below in conjunction with the drawings in the embodiments of the present invention. It is clear that the embodiments described are only partial embodiments of the present invention, and not all of the embodiments. The assembly in embodiments of the present invention generally described and shown in the drawings herein can be arranged and designed in various different configurations. Therefore, the following detailed description of the embodiments of the present invention arranged in the drawings is not intended to limit the scope of the present invention for which protection is claimed, but only represents selected embodiments of the present invention. Based on the embodiments in the present invention, all other embodiments obtained by a person of skill in the art without inventive efforts shall fall within the scope of protection of the present invention.

In the present invention, orientations or positional relationships indicated by the terms "up", "down", "left", "right", "front", "back", "top", "bottom", "inside", "outside", "center", "vertical", "horizontal", "transverse", "longitudinal", etc., are the orientations or positional relationships based on showing in the drawings. These terms are used primarily to better describe the present invention and the embodiments, and are not intended to limit that the indicated device, component, or composition must have a particular orientation, or be constructed and operated in a particular orientation.

Moreover, some of the above terms may be used to indicate other meanings in addition to the orientations or positional relationships, for example, the term "on" may also be used to indicate a certain dependency relationship or a connection relationship in some cases. For a person of ordinary skill in the art, the specific meaning of these terms in the present invention can be understood according to specific situations.

Additionally, the terms "mount", "arrange", "provide", "connect", and "link" are to be understood in a broad sense. For example, it can be a fixed connection, a detachable connection, or an integral construction; it can be a mechanical connection or a point connection; and it can be a direct connection, an indirect connection through an intermediate medium, or a communication inside two devices, components, and compositions. For a person of ordinary skill in the art, the specific meaning of the above terms in the present invention can be understood according to specific situations.

Additionally, the terms "first", "second", etc. are used primarily to distinguish between different devices, components, or compositions (wherein the specific types and constructions may be the same or different), and are not intended to indicate or imply the relative importance and the amounts of the indicated devices, components, or compositions. Unless otherwise indicated, "two" means two or more.

In a first aspect, an embodiment of the present invention provides a (artificial) heart valve stent 10, including a supporting body 100 and at least one protrusion branch body 200 connected to the supporting body 100, wherein a flow channel 120 for blood flow is defined by the supporting body 100; and the protrusion branch body 200 extends from the supporting body 100 to an outer side of the flow channel 120 to form a protrusion part 210 that can abut against the heart tissue, and a gap 300 that can accommodate a native heart valve leaflet 20 is formed between the protrusion branch body 200 and the supporting body 100.

In the above embodiment, the (artificial) heart valve stent 10 includes the supporting body 100 and at least one protrusion branch body 200 connected to the supporting body 100, wherein the supporting body 100 is used to support at the original aortic valve, and a flow channel 120 for blood flow is defined at the middle part of the supporting body 100, wherein the protrusion branch body 200 extends from the supporting body 100 to an outer side away from the flow channel 120, and forms the protrusion part 210 abutting against the heart tissue (such as the aortic sinus). When the (artificial) heart valve stent 10 supports at the original aortic valve, the protrusion part 210 abuts against the heart tissue to fix the (artificial) heart valve stent 10, which prevents the displacement of the (artificial) heart valve stent 10 due to the pressure generated by blood on the valve leaflet 20 when the valve leaflet 20 is closed, so as to improve the stability and reliability of the heart valve stent supporting at the original aortic valve, and to improve the service life of the (artificial) heart valve stent 10. Meanwhile, a gap is arranged between the protrusion branch body 200 and the supporting body 100. When the (artificial) heart valve stent 10 is installed at the original aortic valve, the native heart valve leaflet 20 can be accommodated in the gap, so as to prevent the occurrence of the coronary artery blockage caused by the mutual interference between the native heart valve leaflet 20 and the (artificial) heart valve stent 10, so that it helps to improve the safety of the (artificial) heart valve stent 10 when installed.

In some embodiments, the supporting body includes 100 a plurality of supporting units 110, and the flow channel 120 is formed by the plurality of supporting units 110, wherein each supporting unit 110 includes two first supporting bodies 111 for connecting different valve leaflets 20 respectively and at least one second supporting body 112 for connecting the two first supporting bodies 111, and a space that can be connected and covered by a skirt is formed between the two first supporting bodies 111 and the second supporting body 112.

In the above embodiment, the (artificial) heart valve stent 10 includes the plurality of supporting units 110, and the plurality of supporting units 110 are connected to each other and form the flow channel 120 for blood flow. Each supporting unit 110 includes two first supporting bodies 111 and the second supporting body 112 connected to one end of each of the two first supporting bodies 111 respectively, and a space that is connected and covered by the skirt is formed between the second supporting body 112 and two first supporting bodies 111. When the space is connected and covered by the skirt, the blood can only flow through the flow channel 120, so as to avoid the blood from flowing through the peripheral side of the (artificial) heart valve stent 10.

Exemplarily, three supporting units 110 are provided, and the supporting body 100 is formed by the three supporting units 110.

In some embodiments, upstream and downstream directions are defined according to a direction of blood passing through the flow channel 120, wherein the second supporting body 112 is located at the upstream direction of two first supporting bodies 111.

In the above embodiment, the second supporting body 112 is located at the upstream direction of two first supporting bodies 111, i.e., a blood inflow end is formed in a direction where the second supporting body 112 is located, and a blood outflow end is formed in a direction where two first supporting bodies 111 are located, wherein the blood first flows in from the direction where the second supporting body 112 is located, and then flows out from the direction where the first supporting bodies 111 are located.

In some embodiments, two first supporting bodies 111 extend in the downstream direction from two ends of the second supporting body 112 respectively, and the two first supporting bodies 111 are converged and connected.

In the above embodiment, the first ends of two first supporting bodies 111 are connected by two ends of the second supporting body 112 respectively; second ends extend to the downstream direction of the flow channel 120; and then they are converged and connected, so that a closed ring space for connecting and covering the skirt is formed between the foregoing structure and the second supporting body 112, wherein the second ends of two first supporting bodies 111 can be connected by riveted tubes or welding.

In some embodiments, each supporting unit 110 is connected to the protrusion branch body 200, and the protrusion branch body 200 is located between two adjacent supporting units 110 in a circumferential direction of the supporting body 100.

In the above embodiment, a plurality of protrusion branch bodies 200 are provided, and each supporting unit 110 is connected to the protrusion branch body 200. Specifically, the plurality of protrusion branch bodies 200 are arranged at intervals, and are located between two adjacent supporting units 110 in the circumferential direction of the supporting body 100. After the protrusion parts 210 of the plurality of protrusion branch bodies 200 abut against the heart tissue (such as the aortic sinus), it can effectively improve the reliability and stability of the (artificial) heart valve stent 10 after installation.

In some embodiments, each supporting unit 110 is connected to two protrusion branch bodies 200, and two protrusion branch bodies 200 between two adjacent supporting units 110 are connected to each other.

In the above embodiment, two protrusion branch bodies 200 between two adjacent supporting units 110 are connected at one end of the upstream direction of the flow channel 120, i.e., two protrusion parts 210 of two protrusion branch bodies 200 are connected to each other, which helps to improve the reliability and stability when the protrusion parts 210 abut against the heart tissue.

In some embodiments, two protrusion branch bodies 200 between two adjacent supporting units 110 are formed by one braided wire 131.

In the above embodiment, two protrusion branch bodies 200 between two adjacent supporting units 110 are formed integrally by using one braided wire 131, which does not need the secondary connection such as welding or riveting, which helps to improve the production efficiency of the product.

In some embodiments, a connecting ring 400 is formed in the downstream direction of the first supporting body 111.

In the above embodiment, the connecting ring 400 is formed by the first supporting body 111, and the connecting ring 400 is located downstream the flow channel 120 for connecting to a delivery system of the (artificial) heart valve stent 10, so as to realize the delivery and recovery of the (artificial) heart valve stent 10 by the delivery system.

In some embodiments, the connecting ring 400 is formed on each first supporting body 111.

In the above embodiment, a plurality of connecting rings 400 are provided, and the plurality of connecting rings 400 are located in the downstream direction of the flow channel 120. When the plurality of connecting rings 400 are all connected to the delivery system of the (artificial) heart valve stent 10, it helps to improve the reliability of the (artificial) heart valve stent 10 in the delivery process.

In some embodiments, a first riveted knot 114 is arranged on an upstream part of the connecting ring 400, and the connecting ring 400 forms a closed ring by the first riveted knot 114.

In the above embodiment, the first riveted knot 114 is arranged on the upstream part of the connecting ring 400, so that the connecting ring 400 can form the closed ring structure, which facilitates the connection between the connection member of the delivery system and the connecting ring 400, and helps to improve the reliability when the connection member of the delivery system is connected to the connecting ring 400.

In some embodiments, each supporting unit 110 is connected to the adjacent supporting unit 110 by a riveted structure to form the second riveted knot 115, wherein in the second riveted knot 115, a first supporting body 111 and a second supporting body 112 of two adjacent supporting units 110 are arrayed in parallel, and the gap 300 accommodating the native heart valve leaflet 20 is formed between the protrusion branch body 200 and the first supporting body 111 and the second supporting body 112 in the second riveted knot 115.

In the above embodiment, each supporting unit 110 is connected to two adjacent supporting units 110 by the riveted structure, i.e., the joint of two first supporting bodies 111 and the second supporting body 112 of each supporting unit 110 and the joint of two first supporting bodies 111 and the second supporting body 112 of the adjacent supporting unit 110 are connected by the riveted structure, so as form the second riveted knot 115. In the first riveted knot 114, the first supporting body 111 and the second supporting body 112 of two adjacent supporting unit 110 are arrayed in parrel, which helps to improve the reliability of the connection of each supporting unit 110, and improves the aesthetics of the product; and the protrusion branch body 200 and the first supporting body 111 and the second supporting body 112 in the second riveted knot 115 are arranged at intervals, and defines the gap for accommodating the native heart valve leaflet 20.

In some embodiments, two first supporting bodies 111 of each supporting unit 110 are connected by the riveted structure to form a third riveted knot 116.

In the above embodiment, the joints of two first supporting bodies 111 of each supporting unit 110 located downstream the flow channel 120 are connected by a riveted structure such as a riveted tube, so as to form a third riveted knot 116, which effectively ensures the reliability of the connection of one end of two first supporting bodies 111 of each supporting unit 110 located downstream the flow channel 120.

In some embodiments, the protrusion branch body 200 includes continuous protrusion section 220 and connection section 230 in a direction that the blood passes through the flow channel 120, wherein the protrusion section 220 bends and extends in a direction away from the flow channel 120; one end of the connection section 230 is connected to the protrusion section 220; the other end is connected to the supporting body 100; and an angle between the protrusion section 220 and the flow channel 120 in an axial direction is in a range of 1°~150°.

In the above embodiment, the protrusion section 220 is located downstream the flow channel 120; one end of the connection section 230 is connected to the protrusion section 220, and the other end extends to the flow channel 120 in the downstream direction; and finally the foregoing structure is connected to the first supporting body 111 of the supporting body 100. The angle between the protrusion section 220 and the flow channel 120 in the axial direction is set in a range of 1°~150°, so that the protrusion section 220 can correspond to the position of the heart tissue (such as the aortic sinus), so as to abut against the heart tissue, and help to improve the stability then the protrusion section 220 abuts against the heart tissue.

Specifically, a mounting space is defined between the connection section 230 and the first supporting body 111 for passage of the medical device during installation, such as the coronary stent.

In some embodiments, a horizontal distance a between one end of the connection section 230 connected to the protrusion section 220 and the supporting body 100 located upstream the flow channel 120 is set in a range of 1 mm~20 mm.

In the above embodiment, the distance a between one end of the connection section 230 connected to the protrusion section 220 and the supporting body 100 located upstream the flow channel 120 is set in the range of 1 mm~20 mm, On the one hand, it ensures that the protrusion section 220 can abut against the heart tissue, and it prevents the protrusion section 220 from protruding too long to damage other tissue of the heart; and on the other hand, it facilitates that the native heart valve leaflet 20 is accommodated in the gap between the protrusion branch body 200 and the supporting body 100 after abutting against one side of the protrusion section 220 close to the supporting body 100, so as to improve the convenience when the native heart valve leaflet 20 is accommodated in the gap.

In some embodiments, the heart valve stent 10 is woven by at least one elongated material.

In the above embodiment, exemplary, the elongated material can be a memory alloy wire or a nickel-titanium alloy wire. When the (artificial) heart valve stent 10 is woven by using one elongated material, the (artificial) heart valve stent 10 has a higher integrity and is easier to be processed and shaped. When the (artificial) heart valve stent 10 is woven by using a plurality of elongated materials, two connected elongated materials can be fixedly connected by the riveted tube or welding. Additionally, the joint of two connected elongated materials can be fixedly connected by the welding and threaded connection.

In a second aspect, an embodiment of the present invention further provides another heart valve stent 10, wherein the heart valve stent 10 is woven and shaped by at least one elongated material, and a flow channel 120 for blood flow is defined by the heart valve stent 10, wherein the at least one elongated material protrudes towards an outer side of the flow channel 120 to form the protrusion part 210 that can abut against the heart tissue.

The heart valve stent 10 provided by the embodiments of the present invention is used to support at the original aortic valve, wherein the heart valve stent 10 is woven and shaped by at least one elongated material, and the flow channel 120 for blood flow is defined at the middle part of the heart valve stent 10. Exemplary, the elongated material can be the memory alloy wire or the nickel-titanium alloy wire. Through protruding a local part of at least one elongated material to the outer side away from the flow channel 120, the protrusion part 210 abutting against the heart tissue (such as the aortic sinus) is formed. When the heart valve stent 10 supports at the original aortic valve, the protrusion part 210 abuts against the heart tissue to fix the heart valve stent 10, which prevents the displacement of the heart valve stent 10 due to the pressure generated by blood on the valve leaflet 20 when the valve leaflet 20 is closed, so as to improve the stability and reliability of the heart valve stent 10 supporting at the original aortic valve, and to prolong the service life of the heart valve stent 10, which helps to improve the stability of the heart valve stent 10 in non-calcified patients.

In some embodiments, the heart valve stent 10 includes a plurality of supporting units 110, and the flow channel 120 is formed by the plurality of supporting units 110, wherein each supporting unit 110 includes two first supporting bodies 111 for connecting different valve leaflets 20 respectively and a second supporting body 112 for connecting two first supporting bodies 111, and a space that can be connected and covered by the skirt is formed between the two first supporting bodies 111 and the second supporting body 112.

In some embodiments, the heart valve stent 10 includes the plurality of supporting units 110, and the plurality of supporting units 110 are connected to each other and form the flow channel 120, wherein each supporting unit 110 includes two first supporting bodies 111 and the second supporting body 112 connected to one end of each of the two first supporting bodies 111 respectively, and a space that is connected and covered by the skirt is formed between the second supporting body 112 and two first supporting bodies 111. When the space is connected and covered by the skirt, the blood can only flow through the flow channel 120, so as to avoid the blood from flowing through the peripheral side of the heart valve stent 10.

Exemplarily, three supporting units 110 are provided.

In some embodiments, the upstream and downstream directions are defined according to the direction of blood passing through the flow channel 120, wherein the second supporting body 112 is located at the upstream direction of two first supporting bodies 111.

In the above embodiment, the second supporting body 112 is located at the upstream direction of two first supporting bodies 111, i.e., a blood inflow end is formed in a direction where the second supporting body 112 is located, and a blood outflow end is formed by two first supporting bodies 111, wherein the blood first flows in from the direction where the second supporting body 112 is located, and then flows out from the direction where the first supporting bodies 111 are located.

In some embodiments, the two first supporting bodies 111 extend in the downstream direction from two ends of the second supporting body 112 respectively and second ends of the two first supporting bodies 111 are connected.

In the above embodiment, the first ends of two first supporting bodies 111 are connected by two ends of the second supporting body 112 respectively, and extend to the downstream direction of the flow channel 120; and the second ends of the two first supporting bodies 111 are connected, so as to form a closed ring space for connecting and covering the skirt with the second supporting body 112, wherein the second ends of two first supporting bodies 111 can be connected by riveted tubes or welding.

In some embodiments, each supporting unit 110 further includes a third supporting body 113, and a connecting ring 400 located downstream of the heart valve stent 10 is formed by the third supporting body 113.

In the above embodiment, the supporting unit 110 further includes the third supporting body 113, and the connecting ring 400 located downstream of the heart valve stent 10 is formed by the third supporting body 113. The connecting ring 400 is used to connect to the delivery system of the heart valve stent 10 for realizing delivery and recovery of the heart valve stent 10 by the delivery system.

It can be understood that each supporting unit 110 has two third supporting bodies 113, wherein two third supporting bodies 113 are respectively located at one end downstream the heart valve stent 10, and are respectively connected to the second ends of two first supporting bodies 111 after forming the connecting ring 400.

In some embodiments, one end of the third supporting body 113 located downstream the heart valve stent 10 is connected to the first supporting body 111 after forming the connecting ring 400, and the other end located upstream the heart valve stent 10 forms the protrusion part 210 abutting against the heart tissue, wherein the third supporting body 113 and the first supporting body 111 can be integrally connected, or be fixedly connected by riveting or welding.

In some embodiments, each supporting unit 110 includes two third supporting bodies 113, wherein one end of each of the two third supporting bodies 113 is connected to one of the two first supporting bodies 111 respectively, and the other end of each of two third supporting bodies 113 forms the protrusion part 210.

In the above embodiment, each the supporting unit 110 includes two third supporting bodies 113, and the two third supporting bodies 113 are located on two sides of two first supporting bodies 111, wherein first ends of third supporting bodies 113 are respectively connected to one end of each of two first supporting bodies 111 located downstream the flow channel 120, and the other end forms the protrusion part 210 for abutting against the heart tissue.

In some embodiments, one connecting ring 400 is formed at the most downstream part of each third supporting body 113.

In the above embodiment, one connecting ring 400 is formed at the most downstream part of each third supporting body 113, and the connecting ring 400 is used to connect to the connection structure of the delivery system, so that the heart valve stent 10 is delivered to the heart tissue by the delivery mechanism, which improves the stability and reliability of the delivery process.

In some embodiments, the protrusion part 210 of each supporting unit 110 and the protrusion part 210 of the adjacent supporting unit 110 are formed by bending the same elongated material, and two adjacent protrusion parts 210 are continuous.

In the above embodiment, two adjacent protrusion parts 210 in two adjacent supporting units 110 are formed by bending the same elongated material and are continuous, so as to avoid the damage to the heart tissue by the protrusion parts 210 due to the concentrated stress, which can further improve the support strength of the protrusion part 210 and improve the reliability of the installation of the heart valve stent 10.

In some embodiments, each supporting unit 110 is connected to the adjacent supporting unit 110 by the riveted structure to form a first riveted knot 114, wherein in the first riveted knot 114, individual elongated materials are arrayed in parallel, and the two protrusion parts 210 are located at the middle.

In the above embodiment, each supporting unit 110 is connected to two adjacent supporting units 110 by the riveted structure, i.e., the joint of two first supporting bodies 111 and the second supporting body 112 of each supporting unit 110 and the joint of two first supporting bodies 111 and the second supporting body 112 of the adjacent supporting unit 110 are connected by the riveted structure, so that the first riveted knot 114 is formed, and the third supporting body 113 is also connected to the first riveted knot 114. In the first riveted knot 114, individual elongated materials are arrayed in parallel, which helps to improve the reliability of the connection of each supporting unit 110, and improves the aesthetics of the product; and the protrusion part 210 of each supporting unit 110 is located at the middle part of the supporting unit 110, so as to abut against the heart tissue.

In some embodiments, two first supporting bodies 111 of each supporting unit 110 are connected by the riveted structure to form a second riveted knot 115, and the third supporting body 113 is also connected to the second riveted knot 115.

In the above embodiment, the joints of two first supporting bodies 111 of each supporting unit 110 located downstream the flow channel 120 are connected by the riveted structure such as a riveted tube, so as to form the second riveted knot 115, and two third supporting bodies 113 of each supporting unit 110 are also connected to the second riveted knot 115, so as to effectively ensure the reliability of the connection of two first supporting bodies 111 and two third supporting bodies 113 in each supporting unit 110.

In some embodiments, an angle between the protrusion direction of the protrusion part 210 and a direction perpendicular to the axial direction of the flow channel 120 is in a range of 15°~90°.

In the above embodiment, the angle between the plane where the protrusion part 210 is located and the direction perpendicular to the axial direction of the flow channel 120 is in a range of 15°~90°. When the heart valve stent 10 supports at the original aortic valve, the protrusion part 210 can correspond to the position of the heart tissue, which facilitates the abutment against the heart tissue, and helps to improve the stability of the abutment of the protrusion part 210 against the heart tissue.

In some embodiments, in the direction that the blood passes through the flow channel 120, the third supporting body 113 includes continuous protrusion section 220, transition section 240, and connection section 230, wherein the protrusion part 210 is formed by the protrusion section 220; one end of the transition section 240 is connected to the protrusion section 220; the other end bends and extends in a direction away from the flow channel 120, and has an angle in a range of 60°~150° with a direction perpendicular to the axial direction of the flow channel 120; and one end of the connection section 230 is connected to the transition section 240, and the other end is connected to the first supporting body 111.

In the above embodiment, the third supporting body 113 includes the protrusion section 220, the transition section 240, and the connection section 230 sequentially connected in the flow direction of the flow channel 120, wherein the protrusion section 220 protrudes to the outer side of the flow channel 120 to form the protrusion part 210 for abutting against the heart tissue; one end of the transition section 240 is connected to the protrusion section 220; the other end extends and bends in the direction away from the flow channel 120; and the angle between the transition section 240 and direction perpendicular to the axial direction of the flow channel 120 is set in a range of 60°~150°, so as to ensure that the heart valve stent 10 can support and open the original aortic valve, and thus ensure that the blood can flow normally. One end of the connection section 230 is connected to the transition section 240; and the other end bends and extends in the direction close to the first supporting body 111, and is connected to the first supporting body 111 after forming the connecting ring 400 at the most downstream part of the flow channel 120, so that a larger space is defined between the third supporting body 113 and the valve leaflet 20. Therefore, when the patient undergoes the coronary stent installation surgery, the coronary stent can be installed in the space.

In a third aspect, an embodiment of the present invention provides an implantable heart valve stent 10, wherein the heart valve stent 10 includes the plurality of supporting units 110, and the flow channel 120 for blood flow is formed by the plurality of supporting units 110, wherein at least one of the supporting units 110 includes the protrusion branch body 200; the protrusion part 210 that can abut against the heart tissue is formed by that the protrusion branch body 200 protruding in the direction away from the flow channel 120; and the protrusion branch body 200 extends in the upstream direction of the flow channel 120 to form the connecting body 250 that can connect to the skirt.

In the above embodiment, the heart valve stent 10 includes the plurality of supporting units 110, and the plurality of supporting units 110 are connected to each other and form the flow channel 120 for blood flow, wherein at least one supporting unit 110 includes the protrusion branch body 200, and the protrusion part 210 is formed by the protrusion branch body 200 protruding in the direction away from the flow channel 120, wherein the protrusion part 210 is used to abut against the heart tissue. When the heart valve stent 10 supports at the original aortic valve, the protrusion part 210 abuts against the heart tissue, so as to fix the heart valve stent 10, which prevents the displacement of the heart valve stent 10 due to the pressure generated by blood on the valve leaflet 20 when the valve leaflet 20 is closed, so as to improve the stability and reliability of the heart valve stent supporting at the original aortic valve, and to improve the service life of the heart valve stent 10, Meanwhile, the connecting body 250 for connecting the sealing skirt is formed by the branch body of the protrusion part 210 extending upstream the flow channel 120. When the sealing skirt is connected to the connecting body 250, it can prevent blood from flowing through at the peripheral side of the flow channel 120, which improves the smoothness of the blood when flowing through in the axial direction of the flow channel 120.

Optionally, for the patient with a severely calcified valve leaflet 20, the protrusion part 210 can directly abut against the calcified valve leaflet 20 of the patient. Since the calcified valve leaflet 20 has larger hardness, it can provide a good support effect when abutting against the protrusion part 210, so as to ensure the reliability and stability of the heart valve when supporting at the original heart valve.

In some embodiments, each supporting unit 110 includes two first supporting bodies 111 respectively connecting different valve leaflets 20, and a first space 600-A that can be connected and covered by the skirt is formed between two first supporting bodies 111 and the connecting body 250.

In the above embodiment, each supporting unit 110 includes two first supporting bodies 111, and the first space 600-A connected and covered by the skirt is formed between two first supporting bodies 111 and the connecting body 250. When the space is connected and covered by the skirt, the blood can only flow through the flow channel 120, so as to avoid the blood from flowing through the peripheral side of the heart valve stent 10.

In some embodiments, the upstream and downstream directions are defined according to the direction of blood passing through the flow channel 120, wherein the connecting body 250 is located at the upstream direction of the two first supporting bodies 111, and is continuous to one end of the protrusion branch body 200 located in the upstream direction of the flow channel 120, so as to form the connecting body 250.

In the above embodiment, the connecting body 250 is located in the upstream direction of two first supporting bodies 111, i.e., the blood inflow end is formed in a direction where the connecting body 250 is located, and the blood outflow end is formed in the direction where two first supporting bodies 111 are located, wherein the blood first flows in from the direction where the connecting body 250 is located, and then flows out from the direction where the first supporting bodies 111 are located. The connecting body 250 is continuous with one end of the protrusion branch body 200 located in the upstream direction of the flow channel 120, so that the connecting body 250 connected and covered by the skirt is formed.

In some embodiments, the connecting body 250 includes at least one sub-connecting body 251, and the at least one sub-connecting body 251 and the connecting body 250 are stacked or arranged at intervals.

In the above embodiment, the connecting body 250 is connected to at least one sub-connecting body 251, and the at least two supporting bodies are stacked or arranged at intervals, so that the supporting strength when the connecting body 250 supports at the original heart valve can both be improved by the foregoing two structures, so as to further improve the stability of the heart valve stent 10 when installed.

In some embodiments, two first supporting bodies 111 extend in the downstream direction from two ends of the connecting body 250 respectively, and the two first supporting bodies 111 are converged and connected.

In the above embodiment, the first ends of two first supporting bodies 111 are connected to two ends of the connecting body 250 respectively; and the second ends extend to the downstream direction of the flow channel 120; and then they are converged and connected, so that the closed ring space for connecting and covering the skirt is formed between the foregoing structure and the connecting body 250.

Exemplarily, the connection method between the second ends of two first supporting bodies 111, and the connection method between two first supporting bodies 111 and the connecting body 250 can adopt the riveted tube or welding.

In some embodiments, each supporting unit 110 includes the protrusion branch body 200, and the protrusion branch body 200 is located between two adjacent supporting units 110 in the circumferential direction of the flow channel 120.

In the above embodiment, the plurality of protrusion branch bodies 200 are provided, and each supporting unit 110 includes the protrusion branch body 200. Specifically, the plurality of protrusion branch bodies 200 are arranged at intervals, and are located between two adjacent supporting units 110 in the circumferential direction of the supporting body 100. After the protrusion parts 210 of the plurality of protrusion branch bodies 200 abut against the heart tissue (such as the calcified valve leaflet 20), it can effectively improve the reliability and stability of the heart valve stent 10 after installation.

In some embodiments, each supporting unit 110 includes two protrusion branch bodies 200, and connecting bodies 250 formed by the two protrusion branch bodies 200 are connected to each other.

In the above embodiment, the connecting bodies 250 formed by two protrusion branch bodies 200 of each supporting unit 110 are connected to each other (e.g., integral connection, welding, or riveting), which helps to improve the stability of the sealing skirt connection when connecting and covering in the space formed between the connecting body 250 and the first supporting body 111, and helps to the overall integrity of the product, so as to the reliability and stability of the heart valve stent 10 when supporting at the original heart valve.

In some embodiments, a second space 600-B for the medical device to pass through is formed between each protrusion branch body 200 and the first supporting body 111.

In the above embodiment, the second space 600-B for the medical device such as the coronary stent to pass through is formed between each protrusion branch body 200 and the connected first supporting body 111, so as to facilitate the convenience of installation of the medical device such as a coronary stent after the heart valve stent 10 is installed.

In some embodiments, the protrusion branch body 200 and the first supporting body 111 are woven by one braided wire 131.

In the above embodiment, the protrusion branch body 200 and the first supporting body 111 are woven a single braided wire 131, and the two are continuous, so that it does not need the secondary connection such as welding or riveting, which helps to improve the production efficiency of the product and helps to improve the integrity of the product.

In some embodiments, the connecting ring 400 is formed at one end of the protrusion branch body 200 located downstream the flow channel 120.

In the above embodiment, the protrusion branch body 200 is provided with the connecting ring 400, and the connecting ring 400 is located downstream the flow channel 120 for connecting to the delivery system of the (artificial) heart valve stent 10, so as to realize the delivery and recovery of the heart valve stent 10 by the delivery system.

In some embodiments, each supporting unit 110 is connected to the adjacent supporting unit 110 by the riveted structure to form the first riveted knot 114, wherein in the first riveted knot 114, the first supporting bodies 111 of two adjacent supporting units 110 and the connecting body 250 are arrayed in parallel, and one end of the protrusion branch body 200 located upstream the flow channel 120 is located in the first riveted knot 114.

In the above embodiment, each supporting unit 110 is connected to two adjacent supporting units 110 by the riveted structure, i.e., the joint of two first supporting bodies 111 and the connecting body 250 of each supporting unit 110 and the joint of two first supporting bodies 111 and the connecting body 250 of the adjacent supporting unit 110 are connected together by the riveted structure, so as form the first riveted knot 114. In the first riveted knot 114, the first supporting body 111 and the connecting body 250 of two adjacent supporting unit 110 are arrayed in parrel, which helps to improve the reliability of the connection of each supporting unit 110, and improves the aesthetics of the product; and one end of the protrusion branch body 200 located upstream the flow channel 120 is also located in the second riveted knot 115, and is continuous with the connecting body 250.

In some embodiments, two first supporting bodies 111 of each supporting unit 110 are connected by the riveted structure to form the second riveted knot 115.

In the above embodiment, the joints of two first supporting bodies 111 of each supporting unit 110 located downstream the flow channel 120 are connected by the riveted structure such as a riveted tube, so as to form the second riveted knot 115, which effectively ensures the reliability of the connection of one end of two first supporting bodies 111 of each supporting unit 110 located downstream the flow channel 120.

In some embodiments, the protrusion branch body 200 includes a first connection section 230, an abutting section, and a second connection section 230 connected sequentially, and the first connection section 230 is connected to the first riveted knot 114, wherein a first end of the abutting section is connected to the first connection section 230, and a second end protrudes in a direction away from the flow channel 120; and one end of the second connection section 230 is connected to the second end of the abutting section, and the other end is connected to the first supporting body 111.

In the above embodiment, the protrusion branch body 200 includes the first connection section 230, the abutting section, and the second connection section 230 connected sequentially, wherein at least a part of the first connection section 230 is located in the first riveted knot 114 and is continuous with the connecting body 250; the first end of the abutting section is connected to the first connection section 230, and the second end protrudes to the outer side the flow channel 120, so as to form the protrusion part 210 abutting against the heart tissue; and one end of the second connection section 230 is connected to the abutting section, and the other end extends in the direction close to the flow channel 120, so as to finally connect to the first supporting body 111.

In some embodiments, an angle α between the abutting section and the axis direction of the flow channel 120 is in a range of 10°~150°.

In the above embodiment, the angle α between the abutting section and the axis direction of the flow channel 120 is set in the range of 10°~150°, so that the abutting section abuts against the position of the heart tissue (such as the calcified valve leaflet 20), which helps to improve the stability when the abutting section abuts against the heart tissue.

In some embodiments, a distance b between the second end of the abutting section and the first connection section 230 is in a range of 1 mm~20 mm.

In the above embodiment, the first connection section 230 is connected to the first riveted knot 114 in the axial direction of the flow channel 120, and the distance b between the second end of the abutting section and the first connection section 230 is set in the range of 1 mm~20 mm. On the one hand, it can ensure that the abutting section can abut against the heart tissue; and on the other hand, it can prevent the abutting section from protruding too long towards the outer side of the flow channel 120 to the damage to the heart tissue.

In some embodiments, the implantable heart valve stent 10 is woven by at least one braided wire 131.

In the above embodiment, exemplary, the braided wire 131 can be a memory alloy wire or a nickel-titanium alloy wire. When the heart valve stent 10 is woven by using a braided wire 131, the heart valve stent 10 has a higher integrity and is easier to be processed and shaped. When the heart valve stent 10 is woven by using the plurality of elongated materials, two connected elongated materials can be fixedly connected by the riveted tube or welding. Additionally, the joint of two connected elongated materials can be fixedly connected by the welding and threaded connection.

In some embodiments, the connecting body 250 is woven by a variable-diameter memory alloy wire; or the memory alloy tube 500 is embedded at a part of an outer peripheral side of the connecting body 250.

In the above embodiment, the connecting body 250 is woven by the variable-diameter memory alloy wire braid, or the memory alloy tube 500 is sleeved on a part of the outer peripheral side of the connecting body 250 to locally increase the diameter of the connecting body 250, so that the support force supporting the heart valve stent 10 can be improved, and thus the stability of the support is improved.

Based on the heart valve stent 10 described in the above first aspect and/or second aspect and/or third aspect, in some embodiments, the elongated material or the braided wire 131 includes the memory alloy wire.

Based on the heart valve stent 10 described in the above first aspect and/or second aspect, in the above embodiment, the (artificial) heart valve stent 10 is woven by at least one memory alloy wire, wherein the memory alloy wire can be deformed by the external force, and can recover to its original shape after the external force is withdrawn, which facilitates the delivery of the artificial valve stent by the delivery system after the external force drives the memory alloy wire to deform; and when delivering to the original aortic valve, and the memory alloy wire can recover to its original shape rapidly, which improves the reliability of installation when the heart valve stent 10 supports the aortic valve. When the heart valve stent 10 is woven by the plurality of memory alloy wires, two connected memory alloy wires can be fixedly connected by the riveted tube or welding. Additionally, the heart valve stent 10 can also be fixedly connected by the welding and threaded connection.

Based on the heart valve stent 10 described in the first and/or second aspects above, in some embodiments, the second supporting body 112 is made of at least one variable-diameter memory alloy wire braid; alternatively, a memory alloy tube 500 is locally embedded in the outer peripheral side of the second supporting body 112.

Based on the heart valve stent 10 described in the above first aspect and/or second aspect, in the above embodiment, the second supporting body 112 is woven by the variable-diameter memory alloy wire; or the memory alloy tube 500 can be sleeved on the part of the outer peripheral side of the second supporting body 112 to locally increase the diameter of the second supporting bodies 112, so that the support force supporting the heart valve stent 10 can be improved, and thus the stability of the support is improved.

In a fourth aspect, an embodiment of the present invention provides another heart valve prosthesis 1, including the heart valve stent 10 as described in the first aspect or the second aspect, or the (artificial) heart valve stent 10 of the implantable heart valve stent 10 as described in the third aspect, including: the valve leaflet 20, arranged in the flow channel 120 and connected to the first supporting body 111 of the (artificial) heart valve stent 10; the first sealing skirt 30, connecting and covering in the space formed between two first supporting bodies 111 and the second supporting body 112 of the (artificial) heart valve stent 10; and the second sealing skirt 40, arranged at the outer peripheral side of the (artificial) heart valve stent 10. In some embodiments, the second sealing skirt 40 is arranged at the outer peripheral side of the first sealing skirt 30 and is connected to the first sealing skirt 30 in a sealed method.

In the above embodiment, the valve leaflet 20 is located in the flow channel 120 and is connected to the first supporting body 111 of the heart valve stent 10. The valve leaflets 20 can control whether the blood flows or not by opening or closing, for example, when the heart contracts, the valve leaflets 20 open, so that the blood in the heart flows to the whole body via the aorta; and at the same time, during the diastole of the heart, the valve leaflets 20 can close in time, so as to avoid the blood in the aorta from returning to ventricles. The first sealing skirt 30 is all arranged in the space formed between two first supporting bodies 111 and second supporting bodies 112 of each supporting unit 110 of the heart valve stent 10, so as to avoid the blood from flowing through the peripheral side of the heart valve stent 10, which ensures that the blood flows in only from the blood inflow end and flows out from the blood outflow end. The second sealing skirt 40 is further arranged on the outer peripheral side of the (artificial) heart valve stent 10, wherein the second sealing skirt is used to prevent the blood from returning and to avoid the peripheral leakage.

In some embodiments, the second sealing skirt 40 is in a shape of a disc, and a flange is formed by folding the peripheral side of the second sealing skirt 40 towards downstream the heart valve stent 10.

In the above embodiment, the second sealing skirt 40 is in the shape of the disc. When the heart valve stent 10 supports at the original aortic valve, the second sealing skirt 40 abuts against the original heart valve tissue, and the flange is formed by folding the peripheral side of the second sealing skirt 40 towards downstream direction of the heart valve stent 10. When the valve leaflet 20 is closed, the blood can only flow from the valve leaflet 20 to the upper part of the second sealing skirt 40 and flow through the upper part of the second sealing skirt 40, so as to effectively prevent the blood from returning and to avoid the valve leaflet peripheral leakage.

In some embodiments, the valve leaflet 20 is made of a material including a polymer material, a biological tissue material, and a tissue engineering material.

In the above embodiments, exemplarily, the material of the valve leaflet 20 is a cow pericardium, a pig pericardium, and a cow/pig heart valve, etc.

In some embodiments, the connection method between the valve leaflet 20 and the first supporting body 111 of the (artificial) heart valve stent 10 is one of bonding, heat fusion, and polymer attachment.

In the above embodiment, the valve leaflet 20 can be fixedly connected to the first supporting body 111 of the (artificial) heart valve stent 10 by one method of the bonding, heat fusion, or polymer attachment, so as to avoid the valve leaflet 20 from being damaged or falling off due to stress concentration, which helps to improve the service life of the product.

In a fifth aspect, an embodiment of the present invention further provides another heart valve stent 10, including:
a supporting body 100, including the plurality of supporting units 110, wherein a channel 120 for blood flow is formed by the plurality of supporting units 110; each supporting unit 110 includes two first supporting bodies 111 respectively connecting different valve leaflets 20; at least one of the supporting units 110 includes the second supporting body 112; the second supporting body 112 is arranged on one side of the first supporting body 111 close to the adjacent supporting unit 110; and
at least one protrusion branch body 200, wherein the protrusion branch body 200 is fixed relative to two adjacent supporting units 110 and extends from the supporting body 100 to the outer side of the channel 120, and the gap 300 for accommodating the native heart valve leaflet 20 is formed between the protrusion branch body 200 and the supporting body 100.

The present invention provides the first supporting body 111 and the second supporting body 112 to form the supporting body 100, so that the structure of the supporting body 100 is more stable, and the channel 120 is not easy to deform. The protrusion branch body 200 is arranged to abut against the heart tissue, so that the heart valve stent 10 is not easy to fall off after implanted into the heart, which is more stable. It can prolong the service life of the heart valve stent 10, and reduce the risk of the patient to replace the valve again.

In some embodiments, each supporting unit 110 includes two second supporting bodies 112, and two second supporting bodies 112 in each supporting unit 110 are arranged on two sides of one of the two first supporting bodies 111 respectively.

In the above realization process, two first supporting bodies 111 are provided, and two second supporting bodies 112 are arranged on two sides of each of the two first supporting bodies 111 respectively, so that the overall structure of the supporting body 100 can be more stable.

In some embodiments, each supporting unit 110 includes the third supporting body 113 connecting two first supporting bodies 111, and the space that can be connected and covered by the first skirt 50 is formed between the two first supporting bodies 111 and the third supporting body 113.

In the above realization, the third supporting body 113 is provided, so as to form the space that is covered by the first skirt 50 with the first supporting body 111, which in turn can prevent the blood from returning.

In some embodiments, each first supporting body 111 is fixed relative to one protrusion branch body 200; and each protrusion branch body 200 is fixedly connected relatively to two adjacent first supporting bodies 111 belonging to two adjacent supporting units 110 respectively; and the protrusion part 210 for abutting against the heart tissue is formed at the middle part of the protrusion branch body 200.

In the above realization process, each protrusion branch body 200 is connected to two adjacent first supporting bodies 111, so that the overall structure of the heart valve stent 10 is more stable; and the protrusion part 210 is formed at the middle part of the protrusion branch body 200, so as to abut against the heart tissue, so that the position of the heart valve stent 10 more stable in the heart.

In some embodiments, two adjacent second supporting bodies 112 respectively belonging to two adjacent supporting units 110 are located between protrusion branch bodies 200 connecting the two supporting units 110 in the circumferential direction of the heart valve stent 10.

In the above realization process, two adjacent second supporting bodies 112 respectively belonging to two adjacent supporting units 110 are located between protrusion branch bodies 200 connecting the two supporting units 110 in the circumferential direction of the heart valve stent 10, so that the second supporting bodies 112 can play a better supporting role, so as to make the structure of the heart valve stent 10 more stable.

In some embodiments, two adjacent second supporting bodies 112 respectively belonging to two adjacent supporting units 110 are located at the inner side of the protrusion branch bodies 200 connecting the two supporting units 110 in the radial direction of the heart valve stent 10.

In the above realization process, two adjacent second supporting bodies 112 respectively belonging to two adjacent supporting units 110 are located at the inner side of the protrusion branch bodies 200 connecting the two supporting units 110 in the radial direction of the heart valve stent 10, so that the second supporting bodies 112 can play a better supporting role, so as to make the structure of the heart valve stent 10 more stable. The gap 300 for accommodating the native heart valve leaflet 20 is formed between the protrusion branch body 200 and the supporting body 100, so that the protrusion branch body 200 abuts against the heart tissue, and thus the position of the heart valve stent 10 is more stable in the heart.

In some embodiments, the downstream part of two first supporting bodies 111 of the supporting unit 110 and the downstream part of two protrusion branch bodies 200 are connected by the riveted structure, so as to form the first riveted knot 114, wherein the two protrusion branch bodies 200 are two protrusion branch bodies 200 fixed relative to the two first supporting bodies 111.

In the above realization process, the first supporting body 111 is connected to the protrusion branch body 200 by the riveted structure, which can make the structure of the first supporting body 111 and the protrusion branch body 200 more stable and not easy to deform, so that the structure of the heart valve stent 10 is more stable.

In some embodiments, each second supporting body 112 is fixed relative to one protrusion branch body 200, and the protrusion part 210 for abutting against the heart tissue is formed at the middle part of the protrusion branch body 200.

In the above realization process, each protrusion branch body 200 is connected to one second supporting body 112, so that the overall structure of the heart valve stent 10 is more stable; and the protrusion part 210 is formed at the middle part of the protrusion branch body 200, so as to abut against the heart tissue, so that the position of the heart valve stent 10 is more stable in the heart.

In some embodiments, the parts of two adjacent second supporting bodies 112 respectively belonging to two adjacent supporting units 110 are located between protrusion branch bodies 200 connecting two supporting units 110 in the circumferential direction of the heart valve stent 10.

In the above realization process, the parts of two adjacent second supporting bodies 112 respectively belonging to two adjacent supporting units 110 are located between protrusion branch bodies 200 connecting two supporting units 110 in the circumferential direction of the heart valve stent 10, so that the second supporting bodies 112 can play the better supporting role, so as to make the structure of the heart valve stent 10 more stable.

In some embodiments, the parts of two adjacent second supporting bodies 112 respectively belonging to two adjacent supporting units 110 are located at the inner side of the protrusion branch bodies 200 connecting two supporting units 110 in the radial direction of the heart valve stent 10.

In the above realization process, the parts of two adjacent second supporting bodies 112 respectively belonging to two adjacent supporting units 110 are located at the inner side of the protrusion branch bodies 200 connecting two supporting units 110 in the radial direction of the heart valve stent 10, so that the second supporting bodies 112 can play the better supporting role, so as to make the structure of the heart valve stent 10 more stable; and the gap 300 for accommodating the native heart valve leaflet 20 is formed between the protrusion branch body 200 and the supporting body 100, so that the protrusion branch body 200 abuts against the heart tissue, and thus the position of the heart valve stent 10 is more stable in the heart.

In some embodiments, the downstream parts of two first supporting bodies 111 of the supporting unit 110 are connected by the riveted structure to form the first riveted knot 114; and the downstream part of the protrusion branch body 200 and the second supporting body 112 are connected by the riveted structure to form the second riveted knot 115.

In the above realization process, the two first supporting bodies 111 of the supporting unit 110, the protrusion branch body 200, and the second supporting body 112 are connected by riveted structures respectively, which can make the structure of the first supporting body 111, the protrusion branch body 200, and the second supporting bodies 112 more stable and not easy to deform, so that the structure of the heart valve stent 10 is more stable.

In some embodiments, the upstream parts of two adjacent first supporting bodies 111, upstream parts of two adjacent second supporting bodies 112, and downstream parts of two adjacent third supporting bodies 113 respectively belonging to two adjacent supporting units 110 are connected by riveted structures, so as to form the third riveted knot 116.

In the above realization process, the first supporting bodies 111, the second supporting bodies 112, and the third supporting bodies 113 are connected by riveted structures, which can make the structures of the first supporting bodies 111, the second supporting bodies 112, and the third supporting bodies 113 more stable and not easy to deform, so that the structure of the heart valve stent 10 is more stable.

In some embodiments, each supporting unit 110 is woven by one braided wire 131, and each protrusion branch body 200 is formed by another braided wire 131.

In the above realization process, one supporting unit 110 is woven by one braided wire 131, so as to facilitate the preparation of the supporting unit 110. It does not need other connection structures, which makes the structure of the supporting unit 110 simpler and the structure of the supporting unit 110 solid. The protrusion branch body 200 is formed by the other braided wire 131, so that the gap 300 for accommodating the native heart valve leaflet 20 is formed between the protrusion branch body 200 and the supporting body 100, and thus the protrusion branch body 200 can abut against the heart tissue.

In some embodiments, the braided wire 131 includes a variable-diameter part 134, wherein a diameter of the variable-diameter part 134 is larger than that of the other parts, and the variable-diameter part 134 is arranged corresponding to the riveted structure.

In the above realization process, the variable-diameter part 134 of the braided wire 131 is corresponding to the riveted structure, so as to facilitate the riveting connection of the braided wire 131, so that the braided wire 131 is not easy to slide or even fall off in the riveted knot, and the riveting connection is more stable.

In some embodiments, the braided wire 131 is made of the variable-diameter memory alloy wire to form the variable-diameter part 134; or
a memory alloy tube 500 is embedded at the outer periphery of the braided wire 131, wherein the part having the memory alloy tube 500 is the variable-diameter part 134.

In the above realization process, the braided wire 131 is selected as the reduced diameter memory alloy wire and forms the variable-diameter part 134, which can simplify the installation process of the riveted structure. The memory alloy tube 500 is embedded at the outer periphery of the braided wire 131 to form the variable-diameter part 134, which can simplify the braiding preparation process of the supporting unit 110 or the protrusion branch body 200.

In some embodiments, the connecting ring 400 is formed in the downstream direction of each supporting unit 110.

In the above realization process, the connecting ring 400 is formed in the downstream direction of each supporting unit 110, so that the supporting unit 110 can be woven by one braided wire 131, and the structure of the supporting unit 110 is more stable.

In a sixth aspect, an embodiment of the present invention provides another heart valve prosthesis 1, including the above heart valve stent 10, including
the valve leaflet 20, arranged in the channel 120 and connected to the first supporting body 111 of the heart valve stent 10; and
the first skirt 50, connecting and covering the space formed between two first supporting bodies 111 and the third supporting body 113 of the supporting unit 110 of the heart valve stent 10.

In the above realization process, the first supporting body 111 and the second supporting body 112 are provided to form the supporting body 100, so that the structure of the supporting body 100 is more stable, and the channel 120 is not easy to deform. The protrusion branch body 200 is arranged to abut against the heart tissue, so that the heart valve stent 10 is not easy to fall off after implanted into the heart and is more stable, which can prolong the service life of the heart valve stent 10, and reduce the risk of the patient to replace the valve again. By providing the valve leaflet 20 and the first skirt 50, the blood can flow form the upstream part to the downstream part of the supporting body 100, and it will flow back.

In some embodiments, the heart valve prosthesis 1 further includes the second skirt 60, wherein the second skirt 60 is arranged around the outer peripheral side of the heart valve stent 10; the upstream end of the second skirt 60 is connected to the first skirt 50; and a circumference of the second skirt 60 from the upstream end to the downstream end gradually becomes larger and then gradually becomes smaller.

In the above realization process, the second skirt 60 is arranged around the outer peripheral side of the heart valve stent 10, so that the second skirt 60 can abut against the heart tissue, which further avoids the blood from flowing back. The circumference of the second skirt 60 from the upstream end to the downstream end gradually becomes larger and then gradually becomes smaller, so that the second skirt 60 can abut against the heart tissue at the middle part of the outer periphery, so as to better prevent the blood from flowing back.

In some embodiments, an annular first flange 601 is formed on the downstream end of the second skirt 60, and the first flange 601 faces downstream the heart valve stent 10.

In the above realization process, the first flange 601 is arranged at the downstream end of the second skirt 60, which further improves the effect of preventing the blood from flowing back.

In some embodiments, an accommodation notch 602 is formed on the first flange 601 and the second skirt 60, and the accommodation notch 602 is configured to accommodate the supporting body 100.

In the above realization process, the accommodation notch 602 is formed on the first flange 601 and the second skirt 60, so that the supporting body 100 can be accommodated, and thus the first flange 601 and the second skirt 60 attach with the supporting body 100 better, so to avoid the backflow of the blood from the first flange 601 and the second skirt 60 to the supporting body 100.

In some embodiments, an annular second flange 703 is formed at the peripheral side of the second skirt 60, and the second flange 703 faces downstream the heart valve stent 10.

In the above realization process, the second flange 703 is arranged at the peripheral side of the second skirt 60, which further improves the effect of preventing the blood from flowing back.

In some embodiments, the second flange 703 is arranged on a peripheral side of the largest circumference of the second skirt 60.

In the above realization process, the second flange 703 is arranged on the peripheral side of the largest circumference of the second skirt 60, so that the second flange 703 can abut against the heart tissue, so that the effect of preventing the blood from flowing back is better.

The technical solutions of the present invention have the following effects.
1. The present invention provides at least one protrusion branch body connecting the supporting body, wherein the protrusion branch body protrudes towards the outer side of the flow channel from the supporting body to form the protrusion part, and the protrusion part abuts against the heart tissue, which can better reduce the risk of the displacement of the heart valve stent in patients with pure regurgitation.
2. In the present invention, the gap for accommodating the native heart valve leaflet is formed between the protrusion part and the supporting body, and the native heart valve leaflet is accommodated within the gap, so as to reduce the risk of coronary artery blockage caused by the installation of the heart valve stent.
3. The heart valve stent of the present invention is woven by the memory alloy wire, and the connecting ring for connecting the delivery system is formed, so as to realize the full recovery of the heart valve stent.
4. The heart valve stent of the present invention has a longer service life.
5. The valve leaflet of the present invention is made of polymer material, which helps to improve the service life of the valve leaflet.
6. The heart valve prosthesis of the present invention has a smaller volume, so that the tendency generating the biological incompatibility is smaller.
7. The valve leaflet of the present invention is uniformly coated on the surface of the heart valve stent by using the polymer material, which has a larger adhesion force, so as to avoid situations of damage and detachment of the valve leaflet due to excessive stress when stitching with stitching wires.

Other structures and advantages of the present invention will be described in the subsequent specification, or, partial structures and advantages can be deduced or determined from the specification without doubt, or can be known by performing techniques of the present invention as described above.

In order to make the above purposes, structures, and advantages of the present invention more obvious and easy to understand, the following is a detailed description of preferred embodiments in conjunction with the drawings.

Referring to FIG. 1, FIG. 2, and FIG. 6, an embodiment of the present invention provides a (artificial) heart valve stent 10, including the supporting body 100 and at least one protrusion branch body 200 connected to the supporting body 100, wherein the flow channel 120 for blood flow is defined by the supporting body 100; and the protrusion branch body 200 extends from the supporting body 100 to the outer side of the flow channel 120 to form the protrusion part 210 that can abut against the heart tissue, and the gap 300 that can accommodate the native heart valve leaflet is formed between the protrusion branch body 200 and the supporting body 100.

In the above embodiment, the (artificial) heart valve stent 10 includes the supporting body 100 and at least one protrusion branch body 200 connected to the supporting body 100, wherein the supporting body 100 is used to abut against the original aortic valve, and the flow channel 120 for blood flow is defined at the middle part of the supporting body 100, wherein the protrusion branch body 200 extends from the supporting body 100 to the outer side away from the flow channel 120, and forms the protrusion part 210 abutting against the heart tissue (such as the aortic sinus). When the heart valve stent supports at the original aortic valve, the protrusion part 210 abuts against the heart tissue to fix the heart valve stent, which prevents the displacement of the heart valve stent due to the pressure generated by blood on the valve leaflet 20 when the valve leaflet 20 is closed, so as to improve the stability and reliability of the heart valve stent supporting at the original aortic valve, and to improve the service life of the heart valve stent. Meanwhile, the gap is arranged between the protrusion branch body 200 and the supporting body 100. When the heart valve stent is installed at the original aortic valve, the native heart valve leaflet can be accommodated in the gap, so as to prevent the occurrence of the coronary artery blockage caused by the mutual interference between the native heart valve leaflet and the heart valve stent, so that it helps to improve the safety of the (artificial) heart valve stent 10 when installed.

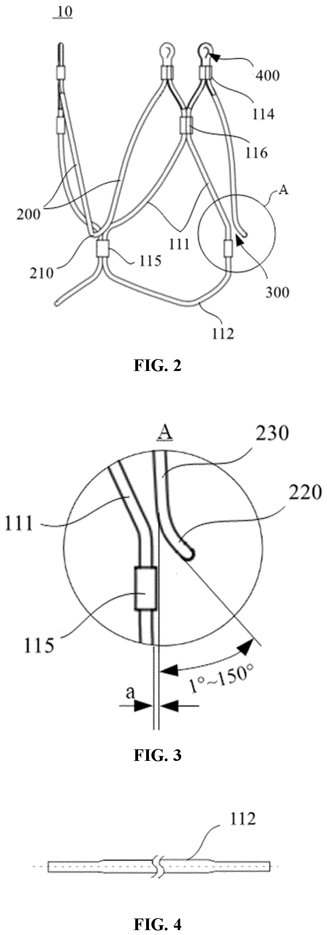

Referring to FIG. 2, in some embodiments, the supporting body includes 100 the plurality of supporting units 110, and the flow channel 120 is formed by the plurality of supporting units 110, wherein each supporting unit 110 includes two first supporting bodies 111 for connecting different valve leaflets 20 respectively and at least one second supporting body 112 for connecting the two first supporting bodies 111, and the space that can be connected and covered by the skirt is formed between the two first supporting bodies 111 and the second supporting body 112.

In the above embodiment, the heart valve stent includes the plurality of supporting units 110, and the plurality of supporting units 110 are connected to each other and form the flow channel 120 for blood flow, wherein each supporting unit 110 includes two first supporting bodies 111 and the second supporting body 112 connected to one end of each of the two first supporting bodies 111 respectively, and the space that is connected and covered by the skirt is formed between the second supporting body 112 and two first supporting bodies 111. When the space is connected and covered by the skirt, the blood can only flow through the flow channel 120, so as to avoid the blood from flowing through the peripheral side of the heart valve stent.

Specifically, in one embodiment, the first supporting body 111 and the second supporting body 112 can be woven by the same braided wire, and they are continuous. The reason for referring to the first supporting body 111 and the second supporting body 112 is for ease of description. Additionally, the supporting unit 110 and the plurality of supporting units 110 can also be woven by the same braided wire.

Exemplarily, three supporting units 110 are provided, and the supporting body 100 is formed by the three supporting units 110.

Referring to FIG. 1 and FIG. 2, in some embodiments, the upstream and downstream directions are defined according to the direction of blood passing through the flow channel 120, wherein the second supporting body 112 is located at the upstream direction of two first supporting bodies 111.

In the above embodiment, the second supporting body 112 is located at the upstream direction of two first supporting bodies 111, i.e., the blood inflow end is formed in the direction where the second supporting body 112 is located, and the blood outflow end is formed in the direction where two first supporting bodies 111 are located, wherein the blood first flows in from the direction where the second supporting body 112 is located, and then flows out from the direction where the first supporting bodies 111 are located.

Referring to FIG. 1 and FIG. 2, in some embodiments, two first supporting bodies 111 extend in the downstream direction from two ends of the second supporting body 112 respectively, and the two first supporting bodies 111 are converged and connected.

In the above embodiment, the first ends of two first supporting bodies 111 are connected by two ends of the second supporting body 112 respectively, second ends extend to the downstream direction of the flow channel 120; and then they are converged and connected, so that the closed ring space for connecting and covering the skirt is formed between the foregoing structure and the second supporting body 112, wherein the second ends of two first supporting bodies 111 can be connected by riveted tubes or welding.

Referring to FIG. 1 and FIG. 2, in some embodiments, each supporting unit 110 is connected to the protrusion branch body 200, and the protrusion branch body 200 is located between adjacent two supporting units 110 in the circumferential direction of the supporting body 100.

In the above embodiment, the plurality of protrusion branch bodies 200 are provided, and each supporting unit 110 is connected to the protrusion branch body 200. Specifically, the plurality of protrusion branch bodies 200 are arranged at intervals, and are located between two adjacent supporting units 110 in the circumferential direction of the supporting body 100. After the protrusion parts 210 of the plurality of protrusion branch bodies 200 abut against the heart tissue (such as the aortic sinus), it can effectively improve the reliability and stability of the (artificial) heart valve stent 10 after installation.

Specifically, in one embodiment, the protrusion branch body 200 and the supporting unit 110 can be woven by the same braided wire, and the protrusion branch body 200 and the supporting unit 110 are continuous.

Referring to FIG. 1 and FIG. 2, in some embodiments, each supporting unit 110 is connected to two protrusion branch bodies 200, and two protrusion branch bodies 200 between two adjacent supporting units 110 are connected to each other.

In the above embodiment, two protrusion branch bodies 200 between two adjacent supporting units 110 are connected at one end of the upstream direction of the flow channel 120, i.e., two protrusion parts 210 of two protrusion branch bodies 200 are connected to each other, which helps to improve the reliability and stability when the protrusion parts 210 abut against the heart tissue.

Referring to FIG. 1 and FIG. 2, in some embodiments, two protrusion branch bodies 200 between two adjacent supporting units 110 are formed by one braided wire.

In the above embodiment, two protrusion branch bodies 200 between two adjacent supporting units 110 are formed integrally by using one braided wire, so that it does not need the secondary connection such as welding or riveting, which helps to improve the production efficiency of the product.

Referring to FIG. 1 and FIG. 2, in some embodiments, the connecting ring 400 is formed in the downstream direction of the first supporting body 111.

In the above embodiment, the connecting ring 400 is formed by the first supporting body 111, wherein the connecting ring 400 is located downstream the flow channel 120 for connecting to the delivery system of the (artificial) heart valve stent 10, so as to realize the delivery and recovery of the heart valve stent by the delivery system.

Referring to FIG. 1 and FIG. 2, in some embodiments, each first supporting body 111 forms the connecting ring 400.

In the above embodiment, the plurality of connecting rings 400 are provided, and the plurality of connecting rings 400 are located in the downstream direction of the flow channel 120. When the plurality of connecting rings 400 are all connected to the delivery system of the (artificial) heart valve stent 10, it helps to improve the reliability of the (artificial) heart valve stent 10 in the delivery process.

Referring to FIG. 1 and FIG. 2, in some embodiments, the first riveted knot 114 is arranged on the upstream part of the connecting ring 400, and the connecting ring 400 forms the closed ring by the first riveted knot 114.

In the above embodiment, the first riveted knot 114 is arranged on the upstream part of the connecting ring 400, so that the connecting ring 400 can form the closed ring structure, which facilitates the connection between the connection member of the delivery system and the connecting ring 400, and helps to improve the reliability when the connection member of the delivery system is connected to the connecting ring 400.

Referring to FIG. 1 and FIG. 2, in some embodiments, each supporting unit 110 is connected to the adjacent supporting unit 110 by the riveted structure to form the second riveted knot 115, wherein in the second riveted knot 115, the first supporting body 111 and the second supporting body 112 of two adjacent supporting units 110 are arrayed in parallel, and the gap 300 accommodating the native heart valve leaflet is formed between the protrusion branch body 200 and the first supporting body 111 and the second supporting body 112 in the second riveted knot 115.

In the above embodiment, each supporting unit 110 is connected to two adjacent supporting units 110 by the riveted structure, i.e., the joint of two first supporting bodies 111 and the second supporting body 112 of each supporting unit 110 and the joint of two first supporting bodies 111 and the second supporting body 112 of the adjacent supporting unit 110 are connected by the riveted structure, so as form the second riveted knot 115. In the first riveted knot 114, the first supporting body 111 and the second supporting body 112 of two adjacent supporting unit 110 are arrayed in parrel, which helps to improve the reliability of the connection of each supporting unit 110, and improves the aesthetics of the product; and the protrusion branch body and the first supporting body 111 and the second supporting body 112 in the second riveted knot 115 are arranged at intervals, and defines the gap for accommodating the native heart valve leaflet.

Referring to FIG. 1 and FIG. 2, in some embodiments, two first supporting bodies 111 of each supporting unit 110 are connected by the riveted structure to form the third riveted knot 116.

In the above embodiment, the joints of two first supporting bodies 111 of each supporting unit 110 located downstream the flow channel 120 are connected by the riveted structure such as a riveted tube, so as to form a third riveted knot 116, which effectively ensures the reliability of the connection of one end of two first supporting bodies 111 of each supporting unit 110 located downstream the flow channel 120.

Referring to FIG. 1 to FIG. 3, in some embodiments, the protrusion branch body 200 includes the continuous protrusion section 220 and the connection section 230 in the direction that the blood passes through the flow channel 120, wherein the protrusion section 220 bends and extends in a direction away from the flow channel 120; one end of the connection section 230 is connected to the protrusion section 220; the other end is connected to the supporting body 100; and the angle between the protrusion section 220 and the flow channel 120 in the axial direction is in the range of 1°~150°.

In the above embodiment, the protrusion section 220 is located downstream the flow channel 120; one end of the connection section 230 is connected to the protrusion section 220, and the other end extends to the flow channel 120 in the downstream direction; and finally the foregoing structure is connected to the first supporting body 111 of the supporting body 100. The angle between the protrusion section 220 and the flow channel 120 in the axial direction is set in a range of 1°~150°, so that the protrusion section 220 can correspond to the position of the heart tissue (such as the aortic sinus), so as to abut against the heart tissue, and help to improve the stability then the protrusion section 220 abuts against the heart tissue.

Specifically, a mounting space is defined between the connection section 230 and the first supporting body 111 for passage of the medical device during installation, such as the coronary stent.

Referring to FIG. 1 to FIG. 3, in some embodiments, the horizontal distance a between one end of the connection section 230 connected to the protrusion section 220 and the supporting body 100 located upstream the flow channel 120 is set in the range of 1 mm~20 mm.

In the above embodiment, the distance a between one end of the connection section 230 connected to the protrusion section 220 and the supporting body 100 located upstream the flow channel 120 is set in the range of 1 mm~20 mm. On the one hand, it ensures that the protrusion section 220 can abut against the heart tissue; and on the other hand, it facilitates that the native heart valve leaflet is accommodated in the gap between the protrusion branch body 200 and the supporting body 100 after abutting against one side of the protrusion section 220 close to the supporting body 100, so as to improve the convenience when the native heart valve leaflet is accommodated in the gap.

In some embodiments, the heart valve stent is woven by at least one elongated material.

In the above embodiment, exemplary, the elongated material can be the memory alloy wire or the nickel-titanium alloy wire. When the (artificial) heart valve stent 10 is woven by using one elongated material, the (artificial) heart valve stent 10 has a higher integrity and is easier to be processed and shaped. When the heart valve stent is woven by using the plurality of elongated materials, two connected elongated materials can be fixedly connected by the riveted tube or welding. Additionally, the joint of two connected elongated materials can be fixedly connected by the welding and threaded connection.

In some embodiments, the elongated material includes the memory alloy wire.

In the above embodiment, the (artificial) heart valve stent 10 is woven by at least one memory alloy wire, wherein the memory alloy wire can be deformed by the external force, and can recover to its original shape after the external force is withdrawn, which facilitates the delivery of the artificial valve stent by the delivery system after the external force drives the memory alloy wire to deform; and when delivering to the original aortic valve, and the memory alloy wire can recover to its original shape rapidly, which improves the reliability of installation when the heart valve stent supports the aortic valve. When the (artificial) heart valve stent 10 is woven by the plurality of memory alloy wires, two connected memory alloy wires can be fixedly connected by the riveted tube or welding. Additionally, the (artificial) heart valve stent 10 can also be fixedly connected by the welding and threaded connection.

Referring to FIG. 4, FIG. 4 is a partial schematic diagram of the second supporting body 112 provided by one embodiment of the present invention. In some embodiments, the second supporting body 112 is woven by at least one variable-diameter memory alloy wire.

Referring to FIG. 5, FIG. 5 is a partial schematic diagram of the second supporting body 112 provided by another embodiment of the present invention. In some embodiments, the memory alloy tube 500 is embedded at a part of an outer peripheral side of the second supporting body 112.

In the above embodiment, the second supporting body 112 is woven by the variable-diameter memory alloy wire; or the memory alloy tube 500 can be sleeved on the part of the outer peripheral side of the second supporting body 112 to locally increase the diameter of the second supporting bodies 112, so that the support force supporting the heart valve stent can be improved, and thus the stability of the support is improved.

Referring to FIG. 6, in a second aspect, the embodiment of the present invention provides a heart valve prosthesis, including the (artificial) heart valve stent 10 according to any one of the embodiments of the first aspect, wherein the valve leaflet 20 is arranged in the flow channel 120 and connected to the first supporting body 111 of the (artificial) heart valve stent 10; the first sealing skirt 30 connects and covers in the space formed between two first supporting bodies 111 and the second supporting body 112 of the (artificial) heart valve stent 10; and the second sealing skirt 40 is arranged at the outer peripheral side of the (artificial) heart valve stent 10.

In the above embodiment, the valve leaflet 20 is located in the flow channel 120 and is connected to the first supporting body 111 of the heart valve stent. The valve leaflets 20 can control whether the blood flows or not by opening or closing, for example, when the heart contracts, the valve leaflets 20 open, so that the blood in the heart flows to the whole body via the aorta; and at the same time, during the diastole of the heart, the valve leaflets 20 can close in time, so as to avoid the blood in the aorta from returning to ventricles. The first sealing skirt 30 is all arranged in the space formed between two first supporting bodies 111 and second supporting bodies 112 of each supporting unit 110 of the heart valve stent, so as to avoid the blood from flowing through the peripheral side of the heart valve stent, which ensures that the blood flows in only from the blood inflow end and flows out from the blood outflow end. The second sealing skirt 40 is further arranged on the outer peripheral side of the (artificial) heart valve stent 10, and the second sealing skirt is used to prevent the blood from returning and to avoid the peripheral leakage.

Referring to FIG. 6, in some embodiments, the second sealing skirt 40 is in a shape of the disc, and the flange is formed by folding the peripheral side of the second sealing skirt 40 towards downstream the heart valve stent.

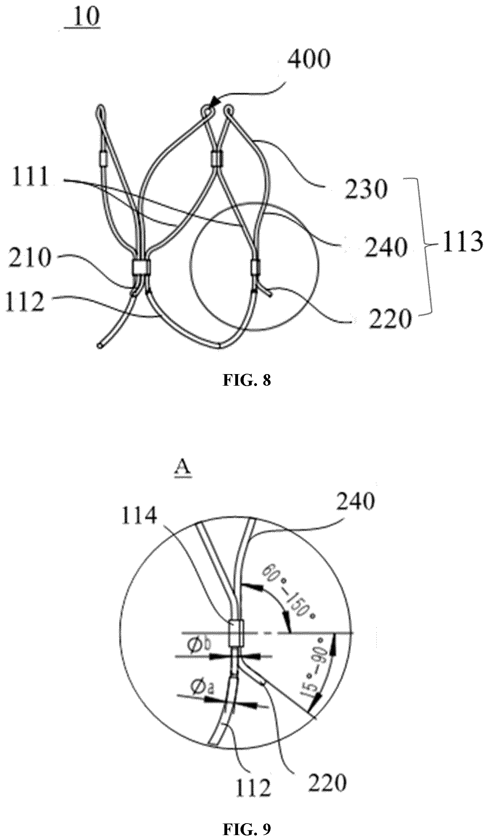

Referring to FIG. 7 to FIG. 9, FIG. 7 shows a structure schematic diagram of the heart valve stent 10 provided by some embodiments of the present invention; FIG. 8 shows a structure schematic diagram of the heart valve stent 10 at the other view provided by some embodiments of the present invention; and FIG. 9 shows an enlarged structure schematic diagram of part A in FIG. 8. The embodiment in the first aspect of the present invention provides a heart valve stent 10, wherein the heart valve stent 10 is woven and shaped by at least one elongated material, and the flow channel 120 for blood flow is defined by the heart valve stent 10, wherein the at least one elongated material protrudes towards the outer side of the flow channel 120 to form the protrusion part 210 that can abut against the heart tissue.

The heart valve stent 10 provided by the embodiments of the present invention is used to support at the original aortic valve, wherein the heart valve stent 10 is woven and shaped by at least one elongated material, and the flow channel 120 for blood flow is defined at the middle part of the heart valve stent 10. Exemplary, the elongated material can be the memory alloy wire or the nickel-titanium alloy wire. Through protruding a local part of at least one elongated material to the outer side away from the flow channel 120, the protrusion part 210 abutting against the heart tissue (such as the aortic sinus) is formed. When the heart valve stent 10 supports at the original aortic valve, the protrusion part 210 abuts against the heart tissue to fix the heart valve stent 10, which prevents the displacement of the heart valve stent 10 due to the pressure generated by blood on the valve leaflet 20 when the valve leaflet 20 is closed, so as to improve the stability and reliability of the heart valve stent 10 supporting at the original aortic valve, and to prolong the service life of the heart valve stent 10, which helps to improve the stability of the heart valve stent 10 in non-calcified patients.

Referring to FIG. 7 and FIG. 8, in some embodiments, the heart valve stent 10 includes the plurality of supporting units 110, and the flow channel 120 is formed by the plurality of supporting units 110, wherein each supporting unit 110 includes two first supporting bodies 111 for connecting different valve leaflets 20 respectively and a second supporting body 112 for connecting two first supporting bodies 111, and a space that can be connected and covered by the skirt is formed between the two first supporting bodies 111 and the second supporting body 112.

Exemplarily, three supporting units are provided.

Referring to FIG. 7 and FIG. 8, in some embodiments, the upstream and downstream directions are defined according to the direction of blood passing through the flow channel 120, wherein the second supporting body 112 is located at the upstream direction of two first supporting bodies 111.

In the above embodiment, the second supporting body 112 is located at the upstream direction of two first supporting bodies 111, i.e., the blood inflow end is formed in the direction where the second supporting body 112 is located, and the blood outflow end is formed by two first supporting bodies 111, wherein the blood first flows in from the direction where the second supporting body 112 is located, and then flows out from the direction where the first supporting bodies 111 are located.

Referring to FIG. 7 and FIG. 8, in some embodiments, the two first supporting bodies 111 extend in the downstream direction from two ends of the second supporting body 112 respectively and second ends of the two first supporting bodies 111 are connected.

In the above embodiment, the first ends of two first supporting bodies 111 are connected by two ends of the second supporting body 112 respectively, and extend to the downstream direction of the flow channel 120, and the second ends of the two first supporting bodies 111 are connected, so as to form the closed ring space for connecting and covering the skirt with the second supporting body 112, wherein the second ends of two first supporting bodies 111 can be connected by riveted tubes or bonding. Additionally, two first supporting bodies 111 and the second supporting body 112 can also be integrally connected, i.e., different parts can be formed after the same elongated material is woven and shaped.

Referring to FIG. 7 and FIG. 8, in some embodiments, each supporting unit 110 further includes the third supporting body 113, and the connecting ring 400 located downstream of the heart valve stent 10 is formed by the third supporting body 113.

In the above embodiment, the supporting unit 110 further includes the third supporting body 113, and the connecting ring 400 located downstream of the heart valve stent 10 is formed by the third supporting body 113. The connecting ring 400 is used to connect to the delivery system of the heart valve stent 10 for realizing delivery and recovery of the heart valve stent 10 by the delivery system.

It can be understood that each supporting unit 110 has two third supporting bodies 113, wherein two third supporting bodies 113 are respectively located at one end downstream the heart valve stent 10, and are respectively connected to the second ends of two first supporting bodies 111 after forming the connecting ring 400. The connection herein includes mechanical connections between two components, such as welding, bonding, and riveting; and it also includes that two components are extensions of the same object, i.e., they are integral, wherein different names refer to different parts.

Referring to FIG. 7 and FIG. 8, in some embodiments, one end of the third supporting body 113 located downstream the heart valve stent 10 is connected to the first supporting body 111 after forming the connecting ring 400, and the other end located upstream the heart valve stent 10 forms the protrusion part 210 abutting against the heart tissue, wherein the third supporting body 113 and the first supporting body 111 can be integrally connected, or be fixedly connected by riveting or welding.

Referring to FIG. 7 and FIG. 8, in some embodiments, each supporting unit 110 includes two third supporting bodies 113, wherein one end of each of the two third supporting bodies 113 is connected to one of the two first supporting bodies 111 respectively, and the other end of each of two third supporting bodies 113 forms the protrusion part 210.

In the above embodiment, each the supporting unit 110 includes two third supporting bodies 113, and the two third supporting bodies 113 are located on two sides of two first supporting bodies 111, wherein first ends of third supporting bodies 113 are respectively connected to one end of each of two first supporting bodies 111 located downstream the flow channel 120, and the other end forms the protrusion part 210 for abutting against the heart tissue.

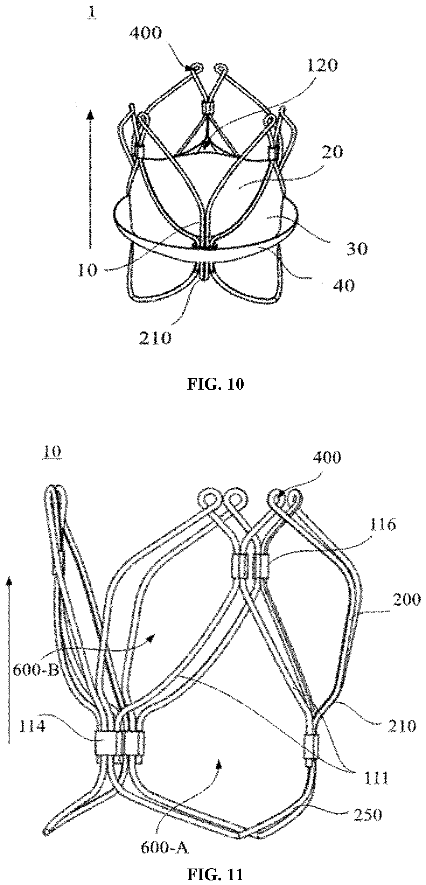

Referring to FIG. 7 and FIG. 10, in some embodiments, one connecting ring 400 is formed at the most downstream part of each third supporting body 113.

In the above embodiment, one connecting ring 400 is formed at the most downstream part of each third supporting body 113, and the connecting rings 400 are all located at the most downstream side of the flow channel 120 for connecting to the connection structure of the delivery system, so that the heart valve stent is delivered to the heart tissue by the delivery mechanism, which improves the stability and reliability of the delivery process.

Referring to FIG. 7 and FIG. 8, in some embodiments, the protrusion part 210 of each supporting unit 110 and the protrusion part 210 of the adjacent supporting unit 110 are formed by bending the same elongated material, and two adjacent protrusion parts 210 are continuous.

In the above embodiment, two adjacent protrusion parts 210 in two adjacent supporting units 110 are formed by bending the same elongated material and are continuous, so as to avoid the damage to the heart tissue by the protrusion parts 210 due to the concentrated stress, which can further improve the support strength of the protrusion part 210 and improve the reliability of the installation of the heart valve stent.

Referring to FIG. 7 and FIG. 8, in some embodiments, each supporting unit 110 is connected to the adjacent supporting unit 110 by the riveted structure to form a first riveted knot 114, wherein in the first riveted knot 114, individual elongated materials are arrayed in parallel, and the two protrusion parts 210 are located at the middle.

In the above embodiment, each supporting unit 110 is connected to two adjacent supporting units 110 by the riveted structure, i.e., the joint of two first supporting bodies 111 and the second supporting body 112 of each supporting unit 110 and the joint of two first supporting bodies 111 and the second supporting body 112 of the adjacent supporting unit 110 are connected by the riveted structure, so that the first riveted knot 114 is formed, and the third supporting body 113 is also connected to the first riveted knot 114. In the first riveted knot 114, individual elongated materials are arrayed in parallel, which helps to improve the reliability of the connection of each supporting unit 110; improves the aesthetics of the product; and also facilitates the contradiction and extension of the heart valve stent 10, and the protrusion part 210 of each supporting unit 110 is located at the middle part of the supporting unit 110, so as to abut against the heart tissue.

Referring to FIG. 7 and FIG. 8, in some embodiments, two first supporting bodies 111 of each supporting unit 110 are connected by the riveted structure to form the second riveted knot 115, and the third supporting body 113 is also connected to the second riveted knot 115.

In the above embodiment, the joints of two first supporting bodies 111 of each supporting unit 110 located downstream the flow channel 120 are connected by the riveted structure such as riveted tubes, so as to form the second riveted knot 115, and two third supporting bodies 113 of each supporting unit 110 are also connected to the second riveted knot 115, so as to effectively ensure the reliability of the connection of two first supporting bodies 111 and two third supporting bodies 113 in each supporting unit 110.

It can be understood that the riveting described in any one of the above embodiments includes a situation that a plurality of components are bound together by a binding member (typically a metal member).

Referring to FIG. 9, in some embodiments, the angle between the plane where the protrusion part 210 is located and the direction perpendicular to the axial direction of the flow channel 120 is in the range of 15°~90°.

In the above embodiment, the angle between the plane where the protrusion part 210 is located and the direction perpendicular to the axial direction of the flow channel 120 is set in the range of 15°~90°. When the heart valve stent 10 supports at the original aortic valve, the protrusion part 210 can correspond to the position of the heart tissue, which facilitates the abutment against the heart tissue, and helps to improve the stability of the abutment of the protrusion part 210 against the heart tissue.

Referring to FIG. 9, in some embodiments, in the direction that the blood passes through the flow channel 120, the third supporting body 113 includes the continuous protrusion section 220, the transition section 240, and the connection section 230, wherein the protrusion part 210 is formed by the protrusion section 220; one end of the transition section 240 is connected to the protrusion section; the other end bends and extends in the direction away from the flow channel 120, and has an angle in a range of 60°~150° with a direction perpendicular to the axial direction of the flow channel 120; and one end of the connection section 230 is connected to the transition section 240, and the other end is connected to the first supporting body 111.

In the above embodiment, the third supporting body 113 includes the protrusion section 220, the transition section 240, and the connection section 230 sequentially connected in the flow direction of the flow channel 120, wherein the protrusion section 220 protrudes to the outer side of the flow channel 120 to form the protrusion part 210 for abutting against the heart tissue; one end of the transition section 240 is connected to the protrusion section 220; the other end extends and bends in the direction away from the flow channel 120; and the angle between the transition section 240 and the direction perpendicular to the axial direction of the flow channel 120 is set in a range of 60°~150°, so as to ensure that the heart valve stent 10 can support and open the original aortic valve, and thus ensure that the blood can flow normally. One end of the connection section 230 is connected to the transition section 240; and the other end bends and extends in the direction close to the first supporting body 111, and is connected to the first supporting body 111 after forming the connecting ring 400 at the most downstream part of the flow channel 120, so that a larger space is defined between the third supporting body 113 and the valve leaflet 20. Therefore, when the patient undergoes the coronary stent installation surgery, the coronary stent can be installed in the space.

Exemplarily, the protrusion section 220, the transition section 240, and the connection section 230 of the third supporting body 113 are integrally connected, i.e., they are woven and shaped by the same elongated material.

Referring to FIG. 6 and FIG. 10, FIG. 10 shows a structure schematic diagram of the heart valve prosthesis 100 provided by some embodiments of the present invention. Based on the structure described in FIG. 6, the heart valve prosthesis 100 further includes the second sealing skirt 40, which is arranged at the outer peripheral side of the first sealing skirt 30 and is connected to the first sealing skirt 30 in a sealed method.

In the above embodiment, the valve leaflet 20 is located in the flow channel 120 and is connected to the heart valve stent 10. The valve leaflets 20 can control whether the blood flows or not by opening or closing, for example, when the heart contracts, the valve leaflets 20 open, so that the blood in the heart flows to the whole body via the aorta; and at the same time, during the diastole of the heart, the valve leaflets 20 can close in time, so as to avoid the blood in the aorta from returning to ventricles. The first sealing skirt 30 is all arranged in the space formed between two first supporting bodies 111 and second supporting bodies 112 of each supporting unit 110 of the heart valve stent 10, so as to avoid the blood from flowing through the peripheral side of the heart valve stent 10, which ensures that the blood flows in only from the blood inflow end and flows out from the blood outflow end. The second sealing skirt 40 connected to the first sealing skirt 30 in a sealed method is further arranged at the outer peripheral side of the first sealing skirt 30, and the second sealing skirt is used to prevent the blood from returning and to avoid the peripheral leakage.

Referring to FIG. 10, in some embodiments, the second sealing skirt 40 is in a shape of the disc, and the flange is formed by folding the peripheral side of the second sealing skirt 40 towards downstream the heart valve stent.

Referring to FIG. 11, FIG. 12, and FIG. 16, the embodiment in the first aspect of the present invention provides an implantable heart valve stent 10, including the plurality of supporting units 110, wherein the flow channel 120 for blood flow is formed by the plurality of supporting units 110, wherein at least one of the supporting units 110 includes the protrusion branch body 200; the protrusion part 210 that can abut against the heart tissue is formed by that the protrusion branch body 200 protruding in the direction away from the flow channel 120; and the protrusion branch body 200 extends in the upstream direction of the flow channel 120 to form the connecting body 250 that can connect to the skirt.

In the above embodiment, the implantable heart valve stent 10 includes the plurality of supporting units 110, and the plurality of supporting units 110 are connected to each other and form the flow channel 120 for blood flow, wherein at least one supporting unit 110 includes the protrusion branch body 200, and the protrusion part 210 is formed by the protrusion branch body 200 protruding in the direction away from the flow channel 120, wherein the protrusion part 210 is used to abut against the heart tissue. When the heart valve stent supports at the original aortic valve, the protrusion part 210 abuts against the heart tissue, so as to fix the heart valve stent, which prevents the displacement of the heart valve stent due to the pressure generated by blood on the valve leaflet 20 when the valve leaflet 20 is closed, so as to improve the stability and reliability of the heart valve stent supporting at the original aortic valve, and to improve the service life of the heart valve stent. Meanwhile, the connecting body 250 for connecting the sealing skirt is formed by the branch body of the protrusion part 210 extending upstream the flow channel 120. When the sealing skirt is connected to the connecting body 250, it can prevent blood from flowing through at the peripheral side of the flow channel 120, which improves the smoothness of the blood when flowing through in the axial direction of the flow channel 120.

Specifically, for the patient with a severely calcified valve leaflet 20, the protrusion part 210 can directly abut against the calcified valve leaflet of the patient. Since the calcified valve leaflet 20 has larger hardness, it can provide a good support effect when abutting against the protrusion part 210, so as to ensure the reliability and stability of the heart valve when supporting at the original heart valve.

Specifically, in one embodiment, the protrusion branch body 200 and the supporting unit 110 can be woven by the same braided wire, and the two are continuous. The reason for referring to the protrusion branch body 200 and the supporting unit 110 is for ease of description. Additionally, each supporting unit 110 and the plurality of supporting units 110 can also be woven by the same braided wire preparation.

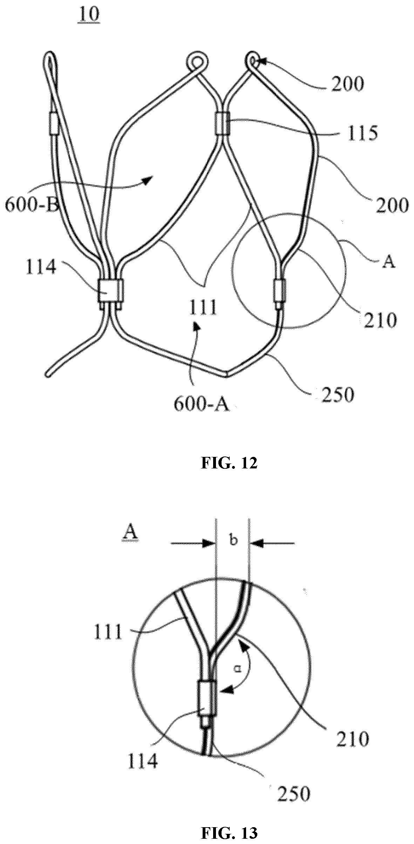

Referring to FIG. 11 and FIG. 12, in some embodiments, each supporting unit 110 includes two first supporting bodies 111 for connecting different valve leaflets 20 respectively and at least one connecting body 250 for connecting two first supporting bodies 111, and the first space 600-A that can be connected and covered by the skirt is formed between the two first supporting bodies 111 and the connecting body 250.

In the above embodiment, each supporting unit 110 includes two first supporting bodies 111, and the first space 600-A that can be connected and covered by the skirt is formed between the two first supporting bodies 111 and the connecting body 250. When the space is connected and covered by the skirt, the blood can only flow through the flow channel 120, so as to avoid the blood from flowing through the peripheral side of the heart valve stent.

Referring to FIG. 11 and FIG. 12, in some embodiments, the upstream and downstream directions are defined according to the direction of blood passing through the flow channel 120, wherein the connecting body 250 is located at the upstream direction of two first supporting bodies 111, and is continuous to one end of the protrusion branch body 200 located in the upstream direction of the flow channel 120, so as to form the connecting body 250.

In the above embodiment, the connecting body 250 is located in the upstream direction of two first supporting bodies 111, i.e., the blood inflow end is formed in the direction where the connecting body 250 is located, and the blood outflow end is formed in the direction where two first supporting bodies 111 are located, wherein the blood first flows in from the direction where the connecting body 250 is located, and then flows out from the direction where the first supporting bodies 111 are located. The connecting body 250 is continuous with one end of the protrusion branch body 200 located in the upstream direction of the flow channel 120, so that the connecting body 250 connected and covered by the skirt is formed.

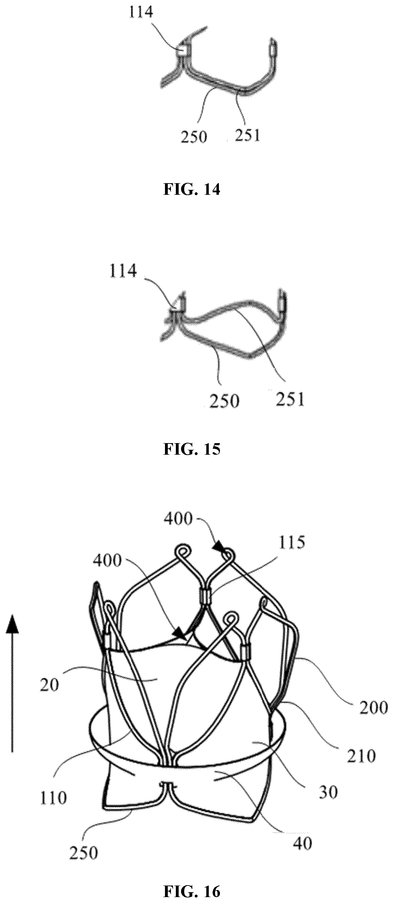

Referring to FIG. 14 and FIG. 15, in some embodiments, the connecting body 250 includes at least one sub-connecting body 251, and the at least one sub-connecting body 251 and the connecting body 250 are stacked or arranged at intervals.

In the above embodiment, the connecting body 250 is connected to at least one sub-connecting body 251, and the at least one sub-connecting body 251 and the connecting body 250 are stacked or arranged at intervals, so that the supporting strength when the connecting body 250 supports at the original heart valve can both be improved by the foregoing two structures, so as to further improve the stability of the heart valve stent when installed.

Referring to FIG. 11 and FIG. 12, in some embodiments, two first supporting bodies 111 extend in the downstream direction from two ends of the connecting body 250 respectively, and the two first supporting bodies 111 are converged and connected.

In the above embodiment, the first ends of two first supporting bodies 111 are connected to two ends of the connecting body 250 respectively; and the second ends extend to the downstream direction of the flow channel 120; and then they are converged and connected, so that the closed ring space for connecting and covering the skirt is formed between the foregoing structure and the connecting body 250.

Exemplarily, the connection method between the second ends of two first supporting bodies 111, and the connection method between two first supporting bodies 111 and the connecting body 250 can adopt the riveted tube or welding.

Referring to FIG. 11 and FIG. 12, in some embodiments, each supporting unit 110 includes the protrusion branch body 200, and the protrusion branch body 200 is located between two adjacent supporting units 110 in the circumferential direction of the flow channel 120.

In the above embodiment, the plurality of protrusion branch bodies 200 are provided, and each supporting unit 110 includes the protrusion branch body 200. Specifically, the plurality of protrusion branch bodies 200 are arranged at intervals, and are located between two adjacent supporting units 110 in the circumferential direction of the supporting body. After the protrusion parts 210 of the plurality of protrusion branch bodies 200 abut against the heart tissue (such as the calcified valve leaflet), it can effectively improve the reliability and stability of the heart valve stent after installation.

Referring to FIG. 11 and FIG. 12, in some embodiments, the connecting bodies 250 formed by two protrusion branch bodies 200 of each supporting unit 110 are connected to each other.

In the above embodiment, the connecting bodies 250 formed by two protrusion branch bodies 200 of each supporting unit 110 are connected to each other (such as the integral connection, welding, or riveting), which helps to improve the stability of the sealing skirt connection when connecting and covering in the space formed between the connecting body 250 and the first supporting body 111, and helps to the overall integrity of the product, so as to the reliability and stability of the heart valve stent when supporting at the original heart valve.

Referring to FIG. 11 and FIG. 12, in some embodiments, the second space 600-B for the medical device to pass through is formed between each protrusion branch body 200 and the first supporting body 111.

In the above embodiment, the second space 600-B for the medical device such as the coronary stent to pass through is formed between each protrusion branch body 200 and the connected first supporting body 111, so as to facilitate the convenience of installation of the medical device such as a coronary stent after the heart valve stent is installed.

In some embodiments, the protrusion branch body 200 and the first supporting body 111 are formed by one braided wire.

In the above embodiment, the protrusion branch body 200 and the first supporting body 111 are woven by one braided wire, and the two are continuous, so that it does not need the secondary connection such as welding or riveting, which helps to improve the production efficiency of the product and helps to improve the integrity of the product.

Referring to FIG. 11, FIG. 12, and FIG. 16, in some embodiments, the connecting ring 400 is formed at one end of the protrusion branch body 200 located downstream the flow channel 120.

In the above embodiment, the protrusion branch body 200 is provided with the connecting ring 400, and the connecting ring 400 is located downstream the flow channel 120 for connecting to the delivery system of the implantable heart valve stent 10, so as to realize the delivery and recovery of the heart valve stent by the delivery system.

Referring to FIG. 11 and FIG. 12, in some embodiments, each supporting unit 110 is connected to the adjacent supporting unit 110 by the riveted structure to form the first riveted knot 114, wherein in the first riveted knot 114, the first supporting body 111 and the connecting body 250 of two adjacent supporting unit 110 are arrayed in parrel, and one end of the protrusion branch body 200 located upstream the flow channel 120 is located in the first riveted knot 114.

In the above embodiment, each supporting unit 110 is connected to two adjacent supporting units 110 by the riveted structure, i.e., the joint of two first supporting bodies 111 and the connecting body 250 of each supporting unit 110 and the joint of two first supporting bodies 111 and the connecting body 250 of the adjacent supporting unit 110 are connected together by the riveted structure, so as form the first riveted knot 114. In the first riveted knot 114, the first supporting body 111 and the connecting body 250 of two adjacent supporting unit 110 are arrayed in parrel, which helps to improve the reliability of the connection of each supporting unit 110, and improves the aesthetics of the product; and one end of the protrusion branch body 200 located upstream the flow channel 120 is also located in the second riveted knot 115, and is continuous with the connecting body 250.

Referring to FIG. 11 and FIG. 12, in some embodiments, two first supporting bodies 111 of each supporting unit 110 are connected by the riveted structure to form the second riveted knot 115.

In the above embodiment, the joints of two first supporting bodies 111 of each supporting unit 110 located downstream the flow channel 120 are connected by the riveted structure such as a riveted tube, so as to form the second riveted knot 115, which effectively ensures the reliability of the connection of one end of two first supporting bodies 111 of each supporting unit 110 located downstream the flow channel 120.

Referring to FIG. 11, FIG. 12, and FIG. 13, in some embodiments, the protrusion branch body 200 includes the first connection section, the abutting section, and the second connection section connected sequentially, and the first connection section is connected to the first riveted knot 114, wherein the first end of the abutting section is connected to the first connection section, and the second end protrudes in the direction away from the flow channel 120; and one end of the second connection section is connected to the second end of the abutting section, and the other end is connected to the first supporting body 111.

In the above embodiment, the protrusion branch body 200 includes the first connection section, the abutting section, and the second connection section connected sequentially, wherein at least a part of the first connection section is located in the first riveted knot 114 and is continuous with the connecting body 250; the first end of the abutting section is connected to the first connection section, and the second end protrudes to the outer side the flow channel 120, so as to form the protrusion part 210 abutting against the heart tissue; and one end of the second connection section is connected to the abutting section, and the other end extends in the direction close to the flow channel 120, so as to finally connect to the first supporting body 111.

Referring to FIG. 13, in some embodiments, the angle α between the abutting section and the axis direction of the flow channel 120 is in a range of 10°~150°.

In the above embodiment, the angle α between the abutting section and the axis direction of the flow channel 120 is set in the range of 10°~150°, so that the abutting section abuts against the position of the heart tissue (such as the calcified valve leaflet), which helps to improve the stability when the abutting section abuts against the heart tissue.

Referring to FIG. 13, in some embodiments, a distance b between the second end of the abutting section and the first connection section is in a range of 1 mm~20 mm.

In the above embodiment, the first connection section is connected to the first riveted knot 114 in the axial direction of the flow channel 120, and the distance b between the second end of the abutting section and the first connection section is set in the range of 1 mm~20 mm. On the one hand, it can ensure that the abutting section can abut against the heart tissue; and on the other hand, it can prevent the abutting section from protruding too long towards the outer side of the flow channel 120 to the damage to the heart tissue.

In some embodiments, the implantable heart valve stent 10 is woven by at least one braided wire.

In the above embodiment, exemplary, the braided wire can be the memory alloy wire or the nickel-titanium alloy wire. When the heart valve stent is woven by using a braided wire, the heart valve stent has a higher integrity and is easier to be processed and shaped. When the heart valve stent is woven by using the plurality of elongated materials, two connected elongated materials can be fixedly connected by the riveted tube or welding. Additionally, the joint of two connected elongated materials can be fixedly connected by the welding and threaded connection.

In some embodiments, the braided wire includes the memory alloy wire.

In the above embodiment, the heart valve stent is woven by at least one memory alloy wire, wherein the memory alloy wire can be deformed by the external force, and can recover to its original shape after the external force is withdrawn, which facilitates the delivery of the implantable heart valve stent 10 by the delivery system after the external force drives the memory alloy wire to deform; and when delivering to the original aortic valve, and the memory alloy wire can recover to its original shape rapidly, which improves the reliability of installation when the heart valve stent supports the aortic valve.

In some embodiments, the connecting body 250 is woven by the variable-diameter memory alloy wire braid; or the memory alloy tube is embedded at the part of an outer peripheral side of the connecting body 250.

In the above embodiment, the connecting body 250 is woven by the variable-diameter memory alloy wire braid, or the memory alloy tube is sleeved on the part of the outer peripheral side of the connecting body 250 to locally increase the diameter of the connecting body 250, so that the support force supporting the heart valve stent can be improved, and thus the stability of the support is improved.

Referring to FIG. 16, the embodiment of the second aspect of the present invention provides a heart valve prosthesis, including the implantable heart valve stent 10 according to any one of embodiments of the first aspect, wherein the valve leaflet 20 is arranged in the flow channel 120 and connected to the first supporting body 111 of the implantable heart valve stent 10; the first sealing skirt 30 connects and covers in the space formed between two first supporting bodies 111 and the connecting body 250 of the heart valve stent; and the second sealing skirt 40 is arranged at the outer peripheral side of the implantable heart valve stent 10.

In the above embodiment, the valve leaflet 20 is located in the flow channel 120 and is connected to the first supporting body 111 of the heart valve stent. The valve leaflets 20 can control whether the blood flows or not by opening or closing, for example, when the heart contracts, the valve leaflets 20 open, so that the blood in the heart flows to the whole body via the aorta; and at the same time, and at the same time, during the diastole of the heart, the valve leaflets 20 can close in time, so as to avoid the blood in the aorta from returning to ventricles. The first sealing skirt 30 is all arranged in the space formed between two first supporting bodies 111 and the connecting body 250 of each supporting unit 110 of the heart valve stent, so as to avoid the blood from flowing through the peripheral side of the heart valve stent, which ensures that the blood flows in only from the blood inflow end and flows out from the blood outflow end. The second sealing skirt 40 is further arranged on the outer peripheral side of the heart valve stent 10, and the second sealing skirt 40 is used to prevent the blood from returning and to avoid the peripheral leakage.

In some embodiments, the connection method between the valve leaflet 20 and the first supporting body 111 of the heart valve stent is one of bonding, heat fusion, and polymer attachment.

In the above embodiment, the second sealing skirt 40 is in the shape of the disc. When the heart valve stent supports at the original aortic valve, the second sealing skirt 40 abuts against the original heart valve tissue, and the flange is formed by folding the peripheral side of the second sealing skirt 40 towards downstream direction of the heart valve stent. When the valve leaflet 20 is closed, the blood can only flow from the valve leaflet 20 to the upper part of the second sealing skirt 40 and flow through the upper part of the second sealing skirt 40, so as to effectively prevent the blood from returning and to avoid the valve leaflet peripheral leakage.

In some embodiments, the valve leaflet 20 is made of the material including the polymer material, biological tissue material, and tissue engineering material.

In the above embodiments, exemplarily, the material of the valve leaflet 20 is a cow pericardium, a pig pericardium, and a cow/pig heart valve, etc.

In some embodiments, the connection method between the valve leaflet 20 and the first supporting body 111 of the (artificial) heart valve stent 10 is one of bonding, heat fusion, and polymer attachment.

In the above embodiment, the valve leaflet 20 can be fixedly connected to the first supporting body 111 of the (artificial) heart valve stent 10 by one method of the bonding, heat fusion, or polymer attachment, so as to avoid the valve leaflet 20 from being damaged or falling off due to stress concentration, which helps to improve the service life of the product.

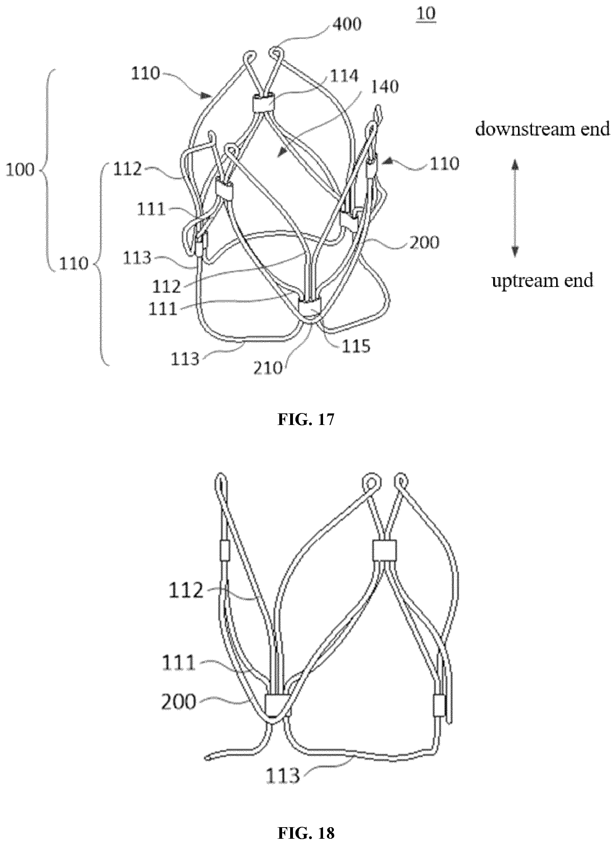

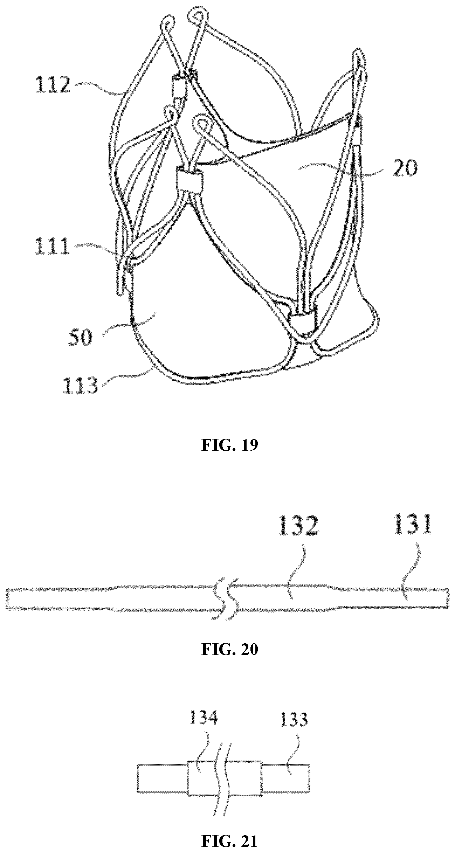

Referring to FIG. 17 to FIG. 19, FIG. 17 shows a schematic diagram of a three-dimensional structure of one heart valve stent provided by embodiments of the present invention; FIG. 18 shows a schematic diagram of a three-dimensional structure of one heart valve stent at the other view provided by embodiments of the present invention; and FIG. 19 shows a schematic diagram of a three-dimensional structure of a heart valve stent, a valve leaflet, and a first skirt provided by embodiments of the present invention. The heart valve stent 10 includes the supporting body 100 and at least one protrusion branch body 200; the supporting body 100 includes the plurality of supporting units 110; and the channel 101 for blood flow is formed by the plurality of supporting units 110. Each supporting unit 110 includes two first supporting bodies 111 respectively connecting different valve leaflets 20; at least one of the supporting units 110 includes the second supporting body 112; the second supporting body 112 is arranged on one side of the first supporting body 111 close to the adjacent supporting unit 110. The protrusion branch body 200 is fixed relative to two adjacent supporting units 110 and extends from the supporting body 100 to the outer side of the channel 101, and the gap (not shown in figures) for accommodating the native heart valve leaflet is formed between the protrusion branch body 200 and the supporting body 100.

The first supporting body 111 and the second supporting body 112 are provided to form the supporting body 100, so that the supporting body 100 can support more parts in the circumferential direction, so that the structure of the supporting body 100 is more stable, and the channel 101 is not easy to deform, wherein the blood can flow more smoothly by the channel 101 without affecting the blood flow rate. The protrusion branch body 200 is arranged to abut against the heart tissue, and the native heart valve leaflet is accommodated between the protrusion branch body 200 and the supporting body 100, so that the heart valve stent 10 is not easy to fall off after implanted into the heart and is more stable, which can prolong the service life of the heart valve stent 10, and reduce the risk of the patient to replace the valve again.

In some embodiments, the supporting body 100 can include three supporting units 110, and the supporting body 100 is formed by the three supporting units 110, wherein the supporting body 100 has a simple structure, and is easy to prepare and relatively stable.

In other embodiments, the supporting body 100 can also be consisted of two supporting units 110 or more than three supporting units 110, which is not limited herein.

In some embodiments, each supporting unit 110 includes two second supporting bodies 112, and two second supporting bodies 112 in each supporting unit 110 are arranged on two sides of one of the two first supporting bodies 111 respectively.

Two first supporting bodies 111 are provided, and two second supporting bodies 112 are arranged on two sides of each of the two first supporting bodies 111 respectively, so that the overall structure of the supporting body 100 can be more stable.

In some embodiments, each supporting unit 110 includes the third supporting body 113 connecting two first supporting bodies 111, and the space that can be connected and covered by the first skirt 50 is formed between the two first supporting bodies 111 and the third supporting body 113.

The third supporting body 113 is provided to form the space that is covered by the first skirt 50 with the first supporting body 111, so as to prevent the blood from returning.

In some embodiments, each first supporting body 111 is fixed relative to one protrusion branch body 200; and each protrusion branch body 200 is fixedly connected relatively to two adjacent first supporting bodies 111 belonging to two adjacent supporting units 110 respectively; and the protrusion part 210 for abutting against the heart tissue is formed at the middle part of the protrusion branch body 200.

Each protrusion branch body 200 is connected to two adjacent first supporting bodies 111, so that two adjacent supporting units 110 can be connected and fixed, and thus the overall structure of the supporting body 100 can be more stable. The protrusion part 210 is formed at the middle part of the protrusion branch body 200, so as to abut against the heart tissue, so that the position of the heart valve stent 10 is more stable in the heart.

In some embodiments, two adjacent second supporting bodies 112 respectively belonging to two adjacent supporting units 110 are located between protrusion branch bodies 200 connecting two supporting units 110 in the circumferential direction of the heart valve stent 10.

Two adjacent second supporting bodies 112 respectively belonging to two adjacent supporting units 110 are located between protrusion branch bodies 200 connecting two supporting units 110 in the circumferential direction of the heart valve stent 10, so that the second supporting bodies 112 can play the better supporting role, which solves the problem of easy deformation due to the larger space between two adjacent first supporting bodies 111 of two supporting units 110, so that the structure of the heart valve stent 10 is more stable.

In some embodiments, two adjacent second supporting bodies 112 respectively belonging to two adjacent supporting units 110 are located at the inner side of the protrusion branch bodies 200 connecting two supporting units 110 in the radial direction of the heart valve stent 10.

Two adjacent second supporting bodies 112 respectively belonging to two adjacent supporting units 110 are located at the inner side of the protrusion branch bodies 200 connecting two supporting units 110 in the radial direction of the heart valve stent 10, so that the second supporting bodies 112 can play the better supporting role, and the structure of the heart valve stent 10 is more stable. Meanwhile, the gap for accommodating the native heart valve leaflet can be formed between the protrusion branch body 200 and the supporting body 100, so that the protrusion branch body 200 abuts against the heart tissue, and thus the position of the heart valve stent 10 is more stable in the heart.

In some embodiments, the downstream part of two first supporting bodies 111 of the supporting unit 110 and the downstream part of two protrusion branch bodies 200 are connected by the riveted structure, so as to form the first riveted knot 114, wherein the two protrusion branch bodies 200 are two protrusion branch bodies 200 fixed relative to the two first supporting bodies 111.

The first supporting body 111 is connected to the protrusion branch body 200 by the riveted structure, which can make the structure of the first supporting body 111 and the protrusion branch body 200 more stable and not easy to deform, so that the structure of the heart valve stent 10 is more stable.

In some embodiments, the upstream parts of two adjacent first supporting bodies 111, upstream parts of two adjacent second supporting bodies 112, and downstream parts of two adjacent third supporting bodies 113 respectively belonging to two adjacent supporting units 110 are connected by riveted structures, so as to form the third riveted knot 116.

The first supporting bodies 111, the second supporting bodies 112, and the third supporting bodies 113 are connected by riveted structures, which can make the structures of the first supporting bodies 111, the second supporting bodies 112, and the third supporting bodies 113 more stable and not easy to deform, so that the structure of the heart valve stent 10 is more stable.

In some embodiments, each supporting unit 110 can be woven by one braided wire 131, and each protrusion branch body 200 can be formed by another braided wire 131.

One supporting unit 110 is woven by one braided wire 131, so as to facilitate the preparation of the supporting unit 110. It does not need other connection structures, which makes the structure of the supporting unit 110 simpler and the structure of the supporting unit 110 solid. The protrusion branch body 200 is formed by the other braided wire 131, and the preparation is simpler, so that the gap for accommodating the native heart valve leaflet is formed between the protrusion branch body 200 and the supporting body 100, and thus the protrusion branch body 200 can abut against the heart tissue.

In some embodiments, the braided wire 131 can include the variable-diameter part, wherein a diameter of the variable-diameter part is larger than that of the other parts, and the variable-diameter part is arranged corresponding to the riveted structure.

The variable-diameter part of the braided wire 131 is corresponding to the riveted structure, which facilitates the riveting connection of the braided wire 131, so that the braided wire 131 is not easy to slide or even fall off in the riveted knot, and the riveting connection is more stable.

In conjunction with FIG. 20, FIG. 20 shows a schematic structure of the braided wire of a heart valve stent provided by embodiments of the present invention. In some embodiments, the braided wire 131 can be made of the variable-diameter memory alloy wire to form the variable-diameter part 132.

The braided wire 131 is selected as the reduced diameter memory alloy wire and forms the variable-diameter part 132; when the supporting unit 110 or the protrusion branch body 200 is woven by the variable-diameter memory alloy wire, the variable-diameter part is directly corresponding to the riveting position; and then the variable-diameter part 132 is connected by the riveting member, which can simplify the installation process of the riveted structure.

In conjunction with FIG. 21, FIG. 21 shows a schematic structure of a braided wire of another heart valve stent provided by embodiments of the present invention. In other embodiments, the memory alloy tube can be embedded at the outer periphery of the braided wire 133, wherein the part having the memory alloy tube is the variable-diameter part 134.

The memory alloy tube is embedded at the outer periphery of the braided wire 133 to form the variable-diameter part 134, so that it does not need to consider the position of the variable-diameter part 134 when the supporting unit 110 or the protrusion branch body 200 is woven by the braided wire 133. After the supporting unit 110 or the protrusion branch body 200 is woven, the memory alloy tube is embedded at the riveting position to form the variable-diameter part 134 for riveting, which can simplify the braiding preparation process of the supporting unit 110.

Referring to FIG. 17 to FIG. 19, in some embodiments, the connecting ring 400 is formed in the downstream direction of each supporting unit 110.

The connecting ring 400 is formed in the downstream direction of each supporting unit 110, so that the supporting unit 110 can be woven by one braided wire 131, and the connecting ring 400 is not easily deformed, which in turn makes the first supporting body 111 and the second supporting body 112 also not easily deformed and the structure of the supporting unit 110 more stable.

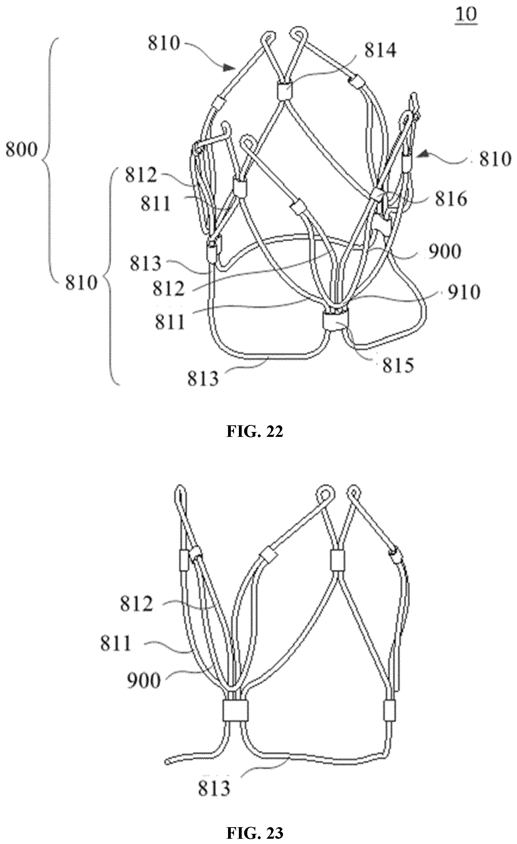

Referring to FIG. 22 and FIG. 23, FIG. 22 shows a schematic diagram of a three-dimensional structure of another heart valve stent provided by embodiments of the present invention; and FIG. 23 shows a schematic diagram of a three-dimensional structure of another heart valve stent at the other view provided by embodiments of the present invention. The heart valve stent 10 includes a supporting body 800 and at least one protrusion branch body 900, wherein the structure of the supporting body 800 is similar to the structure of the supporting body 100 in the above heart valve stent 10, which will not be repeated herein.

The embodiment differs from the above embodiment in that each second supporting body 812 is fixed relative to one protrusion branch body 900, and the protrusion part 910 for abutting against the heart tissue is formed at the middle part of the protrusion branch body 900.

Each protrusion branch body 900 is connected to one second supporting body 812, so that two adjacent supporting units 810 can be connected and fixed, and thus the overall structure of the supporting body 800 can be more stable. The protrusion part 910 is formed at the middle part of the protrusion branch body 900, so as to abut against the heart tissue, so that the position of the heart valve stent 10 is more stable in the heart.

In some embodiments, the parts of two adjacent second supporting bodies 812 respectively belonging to two adjacent supporting units 810 are located between protrusion branch bodies 900 connecting two supporting units 810 in the circumferential direction of the heart valve stent 10.

The parts of two adjacent second supporting bodies 812 respectively belonging to two adjacent supporting units 810 are located between protrusion branch bodies 900 connecting two supporting units 810 in the circumferential direction of the heart valve stent 10, so that the second supporting bodies 812 can play the better supporting role, which solves the problem of easy deformation due to the larger space inside the protrusion branch body 900, so that the structure of the heart valve stent 10 is more stable.

In some embodiments, the parts of two adjacent second supporting bodies 812 respectively belonging to two adjacent supporting units 810 are located at the inner side of the protrusion branch bodies 900 connecting two supporting units 810 in the radial direction of the heart valve stent 10.

The parts of two adjacent second supporting bodies 812 respectively belonging to two adjacent supporting units 810 are located at the inner side of the protrusion branch bodies 900 connecting two supporting units 810 in the radial direction of the heart valve stent 10, so that the second supporting bodies 812 can play the better supporting role, and the structure of the heart valve stent 10 is more stable. Meanwhile, the gap for accommodating the native heart valve leaflet is formed between the protrusion branch body 900 and the supporting body 800, so that the protrusion branch body 900 abuts against the heart tissue, and thus the position of the heart valve stent 10 is more stable in the heart.

In some embodiments, the downstream parts of two first supporting bodies 811 of the supporting unit 810 are connected by the riveted structure to form the first riveted knot 814; and the downstream part of the protrusion branch body 900 and the second supporting body 400 are connected by the riveted structure to form the second riveted knot 815.

The two first supporting bodies 811 of the supporting unit 810, the protrusion branch body 900, and the second supporting body 812 are connected by riveted structures respectively, which can make the structure of the first supporting bodies 811, the protrusion branch body 900, and the second supporting bodies 812 more stable and not easy to deform, so that the structure of the heart valve stent 10 is more stable.

In some embodiments, the upstream parts of two adjacent first supporting bodies 811, upstream parts of two adjacent second supporting bodies 812, and downstream parts of two adjacent third supporting bodies 813 respectively belonging to two adjacent supporting units 810 are connected by riveted structures, so as to form the third riveted knot 816.

The first supporting bodies 811, the second supporting bodies 812, and the third supporting bodies 813 are connected by riveted structures, which can make the structures of the first supporting bodies 811, the second supporting bodies 812, and the third supporting bodies 113 more stable and not easy to deform, so that the structure of the heart valve stent 10 is more stable.

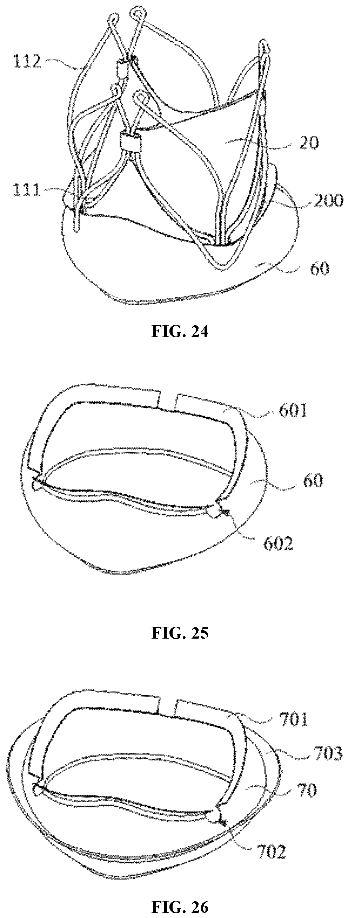

Referring to FIG. 24 and FIG. 19, FIG. 24 shows a structure schematic diagram of a heart valve prosthesis provided by the embodiments of the present invention. The heart valve prosthesis includes the heart valve stent 10, the valve leaflet 20, and the first skirt 50, wherein the valve leaflet 20 is arranged in the channel 101 and connected to the first supporting body 111 of the heart valve stent 10; and the first skirt 50 connects and covers the space formed between two first supporting bodies 111 and the third supporting body 113 of the supporting unit 110 of the heart valve stent 10.

The first supporting body 111 and the second supporting body 112 are provided to form the supporting body 100, so that the structure of the supporting body 100 is more stable, and the channel 101 is not easy to deform. The protrusion branch body 200 is arranged to abut against the heart tissue, so that the heart valve stent 10 is not easy to fall off after implanted into the heart and is more stable, which can prolong the service life of the heart valve stent 10, and reduce the risk of the patient to replace the valve again. By providing the valve leaflet 20 and the first skirt 50, the blood can flow from the upstream part to the downstream part of the supporting body 100, and it will flow back.

In conjunction with FIG. 25, FIG. 25 shows a schematic diagram of a three-dimensional structure of the second skirt in a heart valve prosthesis provided by embodiments of the present invention. In some embodiments, the heart valve prosthesis can further include the second skirt 60, wherein the second skirt 60 is arranged around the outer peripheral side of the heart valve stent 10; the upstream end of the second skirt 60 is connected to the first skirt 50; and the circumference of the second skirt 60 from the upstream end to the downstream end gradually becomes larger and then gradually becomes smaller.

The second skirt 60 is arranged around the outer peripheral side of the heart valve stent 10, so that the second skirt 60 can abut against the heart tissue, which further avoids the blood from flowing back. The circumference of the second skirt 60 from the upstream end to the downstream end gradually becomes larger and then gradually becomes smaller, so that the second skirt 60 can abut against the heart tissue at the middle part of the outer periphery, so as to better prevent the blood from flowing back.

In some embodiments, the annular first flange 601 can be formed on the downstream end of the second skirt 60, and the first flange 601 faces downstream the heart valve stent 10.

The first flange 601 is arranged at the downstream end of the second skirt 60, which further improves the effect of preventing the blood from flowing back.

In some embodiments, the accommodation notch 602 is formed on the first flange 601 and the second skirt 60, and the accommodation notch 602 is configured to accommodate the supporting body 100.

The accommodation notch 602 is formed on the first flange 601 and the second skirt 60, so that the supporting body 100 can be accommodated, and thus the first flange 601 and the second skirt 60 attach with the supporting body 100 better, so to avoid the backflow of the blood from the first flange 601 and the second skirt 60 to the supporting body 100.

In conjunction with FIG. 26, FIG. 26 shows a schematic diagram of a three-dimensional structure of the second skirt in another heart valve prosthesis provided by embodiments of the present invention. In some embodiments, the first flange 701 can be arranged at the downstream end of the second skirt 70; the accommodation notch 702 can be formed on the first flange 701 and the second skirt 70; the annular second flange 703 can further be formed on the peripheral side of the second skirt 70; and the second flange 703 faces downstream the heart valve stent 10.

The second flange 703 is arranged at the peripheral side of the second skirt 70, so that the second flange 703 can abut against the heart tissue, which can further improve the effect of preventing the blood from flowing back.

In some embodiments, the second flange 703 is arranged on the peripheral side of the largest circumference of the second skirt 70.

The second flange 703 is arranged on the peripheral side of the largest circumference of the second skirt 70, so that the second flange 703 can abut against the heart tissue better, and the effect of preventing the blood from flowing back is better.

In other embodiments, the second flange 703 can also arranged between the downstream end of the second skirt 70 and the peripheral side of the largest circumference of the second skirt 70, which is not limited herein.

In all embodiments of the present invention, "large" and "small" are relative, "more" and "less" are relative, "up" and "down" are relative, and the expression of such relative terms is not repeated in the embodiment of the present invention.

It is understood that references throughout the specification to "in the embodiment", "in the embodiment of the present invention" or "in one embodiment" imply that a particular structure, structure, or feature relative to the embodiment is included in at least one embodiment of the present invention. Therefore, "in the embodiment", "in the embodiment of the present invention", or "in one embodiment" appearing throughout the specification may not necessarily refer to the same embodiment. Additionally, these particular structures, structures, or features may be combined in one or two embodiments in any suitable method. It should also be appreciated by those skilled in the art that the embodiments described in the specification are all optional embodiments, and the actions and modules involved are not necessarily necessary for the present invention.

In the various embodiments of the present invention, it should be understood that the magnitude of the sequence number of the processes above does not imply an inevitable sequence of execution. The sequence of execution of each process shall be determined by its function and internal logic, and shall not constitute any limitation on the implementation process of the embodiments of the present invention.

The above is only the preferable embodiment of the present invention, and the scope of protection of the present invention is not limited to this. Any change or replacement that can be easily thought of by any technical person familiar with the technical field within the technical scope disclosed by the present invention shall be covered within the protection scope of the present invention. Therefore, the scope of protection of the present invention shall be governed by the scope of protection of the claims.

It is to be noted that in the text, relationship terms such as first and second, etc., are used merely to distinguish one entity or operation from another, and do not necessarily require or imply the existence of any this actual relationship or order between those entities or operations. Furthermore, the terms "include", "comprise" or any other variations thereof are intended to cover a non-exclusive inclusion, so that a process, method, object, or device including a series of elements includes not only those elements, but also other elements that are not explicitly listed, or the components that are inherent to this process, method, object, or device. Without further limitation, the component defined by a phrase "including a ......" does not exclude that the other same elements also exist in the process, method, object, or device including the component.

### Industrial Applicability

The present invention relates to the technical field of medical devices, and a (implantable) heart valve stent and a heart valve prosthesis are provided. The solutions of the present invention can improve the stability and reliability of installation and fixation of the heart valve stent; improve the service life of the heart valve stent; and reduce the risk of the patient to replace the valve again; and it helps the patient to avoid the coronary artery blockage when using the heart valve stent.

## Claims

1. A heart valve stent, **characterized by** comprising a supporting body and at least one protrusion branch body connected to the supporting body, wherein a flow channel for blood flow is defined by the supporting body; and the protrusion branch body extends from the supporting body to an outer side of the flow channel to form a protrusion part that can abut against a heart tissue, and a gap that can accommodate a native heart valve leaflet is formed between the protrusion branch body and the supporting body.

2. The heart valve stent according to claim 1, wherein the supporting body comprises a plurality of supporting units, and the flow channel is formed by the plurality of supporting units, wherein
each of the supporting units comprises two first supporting bodies for connecting different valve leaflets respectively and a second supporting body connecting the two first supporting bodies, and a space that can be connected and covered by a skirt is formed between the two first supporting bodies and the second supporting body.

3. A heart valve stent, **characterized in that** the heart valve stent is woven and shaped by at least one elongated material, and a flow channel for blood flow is defined by the heart valve stent, wherein the at least one elongated material protrudes towards an outer side of the flow channel to form a protrusion part that can abut against a heart tissue.

4. The heart valve stent according to claim 3, wherein the heart valve stent comprises a plurality of supporting units, and the flow channel is formed by the plurality of supporting units, wherein
each of the supporting units comprises two first supporting bodies for connecting different valve leaflets respectively and a second supporting body connecting the two first supporting bodies, and a space that can be connected and covered by a skirt is formed between the two first supporting bodies and the second supporting body.

5. The heart valve stent according to claim 2 or 4, wherein upstream and downstream directions are defined according to a direction of blood passing through the flow channel, wherein the second supporting body is located at an upstream direction of the two first supporting bodies.

6. The heart valve stent according to claim 5, wherein the two first supporting bodies extend in a downstream direction from two ends of the second supporting body respectively, and the two first supporting bodies are converged and connected;
optionally, the two first supporting bodies extend in the downstream direction from the two ends of the second supporting body respectively and second ends of the two first supporting bodies are connected.

7. The heart valve stent according to any one of claims 2 and 5-6, wherein each of the supporting units is connected to the protrusion branch body, and the protrusion branch body is located between two adjacent supporting units in a circumferential direction of the supporting body.

8. The heart valve stent according to claim 7, wherein each of the supporting units is connected to two protrusion branch bodies, and the two protrusion branch bodies between the two adjacent supporting units are connected to each other.

9. The heart valve stent according to claim 8, wherein the two protrusion branch bodies between the two adjacent supporting units are formed by one braided wire.

10. The heart valve stent according to any one of claims 2 and 5-6, wherein a connecting ring is formed in a downstream direction of the first supporting body.

11. The heart valve stent according to claim 10, wherein the connecting ring is formed on each of the first supporting bodies.

12. The heart valve stent according to claim 10, wherein a first riveted knot is arranged on an upstream part of the connecting ring, and the connecting ring forms a closed ring by the first riveted knot.

13. The heart valve stent according to any one of claims 2 and 5-6, wherein each of the supporting units is connected to an adjacent supporting unit by a riveted structure to form a second riveted knot, wherein in the second riveted knot, a first supporting body and a second supporting body of the two adjacent supporting units are arrayed in parallel, and the gap accommodating the native heart valve leaflet is formed between the protrusion branch body and the first supporting body and the second supporting body located in the second riveted knot.

14. The heart valve stent according to any one of claims 2 and 5-6, wherein the two first supporting bodies of each of the supporting units are connected by a riveted structure to form a third riveted knot.

15. The heart valve stent according to any one of claims 1-2 and 5-6, wherein the protrusion branch body comprises continuous protrusion section and connection section in a direction that the blood passes through the flow channel, wherein
the protrusion section bends and extends in a direction away from the flow channel; one end of the connection section is connected to the protrusion section; the other end is connected to the supporting body; and an angle between the protrusion section and the flow channel in an axial direction is in a range of 1°~150°.

16. The heart valve stent according to claim 15, wherein a horizontal distance a between one end of the connection section connected to the protrusion section and the supporting body located upstream the flow channel is set in a range of 1 mm~20 mm.

17. The heart valve stent according to any one of claims 1-2 and 5-6, wherein the heart valve stent is woven by at least one elongated material.

18. The heart valve stent according to claim 4 or 6, wherein each of the supporting units further comprises a third supporting body, and a connecting ring located downstream the heart valve stent is formed by the third supporting body.

19. The heart valve stent according to claim 18, wherein one end of the third supporting body is connected to the first supporting bodies, and the other end forms the protrusion part.

20. The heart valve stent according to claim 19, wherein each of the supporting units comprises two third supporting bodies, wherein one end of each of the two third supporting bodies is connected to one of the two first supporting bodies respectively, and the other end of each of the two third supporting bodies forms the protrusion part.

21. The heart valve stent according to claim 20, wherein one connecting ring is formed at a most downstream part of each of the third supporting bodies.

22. The heart valve stent according to any one of claims 4-6 and 18-21, wherein a protrusion part of each of the supporting units and a protrusion part of an adjacent supporting unit are formed by bending the same elongated material, and the two adjacent protrusion parts are continuous.

23. The heart valve stent according to any one of claims 4-6 and 18-21, wherein each of the supporting units is connected to an adjacent supporting unit by a riveted structure to form a first riveted knot, wherein in the first riveted knot, individual elongated materials are arrayed in parallel, and the two protrusion parts are located at the middle.

24. The heart valve stent according to any one of claims 18-21, wherein the two first supporting bodies of each of the supporting units are connected by a riveted structure to form a second riveted knot, and the third supporting bodies are also connected to the second riveted knot.

25. The heart valve stent according to any one of claims 3-6 and 18-21, wherein an angle between a protrusion direction of the protrusion part and a direction perpendicular to an axial direction of the flow channel is in a range of 15°~90°.

26. The heart valve stent according to any one of claims 18-21, wherein in a direction that the blood passes through the flow channel, each of the third supporting bodies comprises continuous protrusion section, transition section, and connection section, wherein
the protrusion part is formed by the protrusion section; one end of the transition section is connected to the protrusion section; the other end bends and extends in a direction away from the flow channel, and has an angle in a range of 60°~150° with a direction perpendicular to the axial direction of the flow channel; and one end of the connection section is connected to the transition section, and the other end is connected to the first supporting body.

27. The heart valve stent according to any one of claims 3-4 and 17-26, wherein the elongated material comprises a memory alloy wire.

28. The heart valve stent according to any one of claims 2, 5-6, and 18-26, wherein the second supporting body is woven by a variable-diameter memory alloy wire; or
a memory alloy tube is embedded at a part of an outer peripheral side of the second supporting body.

29. An implantable heart valve stent, **characterized in that**
the implantable heart valve stent comprises a plurality of supporting units, and a flow channel for blood flow is formed by the plurality of supporting units, wherein
at least one of the supporting units comprises a protrusion branch body; a protrusion part that can abut against a heart tissue is formed by the protrusion branch body protruding in a direction away from the flow channel; and the protrusion branch body extends in an upstream direction of the flow channel to form a connecting body that can connect to a skirt.

30. The implantable heart valve stent according to claim 29, wherein each of the supporting units comprises two first supporting bodies respectively connecting different valve leaflets, and a first space that can be connected and covered by the skirt is formed between the two first supporting bodies and the connecting body.

31. The implantable heart valve stent according to claim 30, wherein upstream and downstream directions are defined according to a direction of blood passing through the flow channel, wherein the connecting body is located at an upstream direction of the two first supporting bodies.

32. The implantable heart valve stent according to claim 30, wherein the connecting body comprises at least one sub-connecting body, and the at least one sub-connecting body and the connecting body are stacked or arranged at intervals.

33. The implantable heart valve stent according to any one of claims 30-32, wherein the two first supporting bodies extend in the downstream direction from two ends of the connecting body respectively, and the two first supporting bodies are converged and connected.

34. The implantable heart valve stent according to any one of claims 29-32, wherein each of the supporting units comprises the protrusion branch body, and the protrusion branch body is located between two adjacent supporting units in a circumferential direction of the flow channel.

35. The implantable heart valve stent according to claim 34, wherein each of the supporting units comprises the two protrusion branch bodies, and connecting bodies formed by the two protrusion branch bodies are connected to each other.

36. The implantable heart valve stent according to claim 35, wherein a second space for a medical device to pass through is formed between each of the protrusion branch bodies and the first supporting bodies.

37. The implantable heart valve stent according to any one of claims 30-32, wherein the protrusion branch body and the first supporting bodies are woven by one braided wire.

38. The implantable heart valve stent according to any one of claims 29-32, wherein a connecting ring is formed at one end of the protrusion branch body located downstream the flow channel.

39. The implantable heart valve stent according to any one of claims 30-32, wherein each of the supporting units is connected to an adjacent supporting unit by a riveted structure to form a first riveted knot, wherein in the first riveted knot, the first supporting bodies of the two adjacent supporting units and the connecting body are arrayed in parallel.

40. The implantable heart valve stent according to any one of claims 30-32, wherein the two first supporting bodies of each of the supporting units are connected by a riveted structure to form a second riveted knot.

41. The implantable heart valve stent according to any one of claims 30-32, wherein the protrusion branch body comprises a first connection section, an abutting section, and a second connection section connected sequentially, and the first connection section is connected to the first riveted knot, wherein a first end of the abutting section is connected to the first connection section, and a second end protrudes in a direction away from the flow channel; and one end of the second connection section is connected to the second end of the abutting section, and the other end is connected to the first supporting body.

42. The implantable heart valve stent according to claim 41, wherein an angle α between the abutting section and an axis direction of the flow channel is in a range of 10°~150°.

43. The implantable heart valve stent according to claim 41, wherein a distance b between the second end of the abutting section and the first connection section is in a range of 1 mm~20 mm.

44. The implantable heart valve stent according to any one of claims 29-32, wherein the implantable heart valve stent is woven by at least one braided wire.

45. The implantable heart valve stent according to claim 42, wherein the braided wire comprises a memory alloy wire.

46. The implantable heart valve stent according to any one of claims 30-32, wherein the connecting body is woven by a variable-diameter memory alloy wire; or
a memory alloy tube is embedded at a part of an outer peripheral side of the connecting body.

47. A heart valve prosthesis, **characterized by** comprising the heart valve stent according to any one of claims 1-28 or the implantable heart valve stent according to any one of claims 29-46, comprising:
a valve leaflet, arranged in the flow channel and connected to the first supporting bodies of the heart valve stent or the implantable heart valve stent; and
a first sealing skirt, connecting and covering a space formed between the two first supporting bodies and the second supporting body of the heart valve stent or the implantable heart valve stent;
optionally, a second sealing skirt, formed around an outer peripheral side of the heart valve stent or the implantable heart valve stent.

48. The heart valve prosthesis according to claim 47, wherein the second sealing skirt is in a shape of a disc, and a flange is formed by folding a peripheral side of the second sealing skirt towards downstream the heart valve stent.

49. The heart valve prosthesis according to claim 47 or 48, wherein the valve leaflet is made of a material comprising at least one of a polymer material, a biological tissue material, and a tissue engineering material.

50. The heart valve prosthesis according to claim 47 or 48, wherein a connection method of the valve leaflet and the first supporting body of the heart valve stent or the implantable heart valve stent is one of suture stitching, bonding, heat fusion, and polymer attachment.

51. A heart valve stent, **characterized by** comprising:
a supporting body, comprising a plurality of supporting units, wherein a channel for blood flow is formed by the plurality of supporting units; each of the supporting units comprises two first supporting bodies respectively connecting different valve leaflets; at least one of the supporting units comprises a second supporting body; the second supporting body is arranged on one side of the first supporting bodies close to an adjacent supporting unit; and
at least one protrusion branch body, wherein the protrusion branch body is fixed relative to two adjacent supporting units and extends from the supporting body to an outer side of the channel, and a gap for accommodating the native heart valve leaflet is formed between the protrusion branch body and the supporting body.

52. The heart valve stent according to claim 51, wherein each of the supporting units comprises two second supporting bodies, and the two second supporting bodies in each of the supporting units are arranged on two sides of one of the two first supporting bodies respectively.

53. The heart valve stent according to claim 52, wherein each of the supporting units comprises a third supporting body connecting the two first supporting bodies, and a space that can be connected and covered by a first skirt is formed between the two first supporting bodies and the third supporting body.

54. The heart valve stent according to claim 53, wherein each of the first supporting bodies is fixed relative to one of the protrusion branch bodies; and each of the protrusion branch bodies is fixedly connected relatively to two adjacent first supporting bodies belonging to two adjacent supporting units respectively; and a protrusion part for abutting against a heart tissue is formed at a middle part of the protrusion branch bodies.

55. The heart valve stent according to claim 54, wherein two adjacent second supporting bodies respectively belonging to two adjacent supporting units are located between protrusion branch bodies connecting the two supporting units in a circumferential direction of the heart valve stent.

56. The heart valve stent according to claim 54, wherein two adjacent second supporting bodies respectively belonging to two adjacent supporting units are located at an inner side of the protrusion branch bodies connecting the two supporting units in a radial direction of the heart valve stent.

57. The heart valve stent according to any one of claims 54-56, wherein a downstream part of the two first supporting bodies of the supporting units and a downstream part of the two protrusion branch bodies are connected by a riveted structure, so as to form a first riveted knot, wherein the two protrusion branch bodies are two protrusion branch bodies fixed relative to the two first supporting bodies.

58. The heart valve stent according to claim 53, wherein each of the second supporting bodies is fixed relative to one of the protrusion branch bodies, and a protrusion part for abutting against a heart tissue is formed at the middle part of the protrusion branch bodies.

59. The heart valve stent according to claim 58, wherein parts of two adjacent second supporting bodies respectively belonging to two adjacent supporting units are located between protrusion branch bodies connecting the two supporting units in a circumferential direction of the heart valve stent.

60. The heart valve stent according to claim 58, wherein parts of two adjacent second supporting bodies respectively belonging to two adjacent supporting units are located at an inner side of the protrusion branch bodies connecting the two supporting units in a radial direction of the heart valve stent.

61. The heart valve stent according to any one of claims 58-60, wherein downstream parts of the two first supporting bodies of the supporting units are connected by a riveted structure to form a first riveted knot; and a downstream part of the protrusion branch bodies and the second supporting body are connected by a riveted structure to form a second riveted knot.

62. The heart valve stent according to claim 53, wherein upstream parts of two adjacent first supporting bodies, upstream parts of two adjacent second supporting bodies, and downstream parts of two adjacent third supporting bodies respectively belonging to two adjacent supporting units are connected by a riveted structure, so as to form a third riveted knot.

63. The heart valve stent according to claim 57, 61, or 62, wherein each of the supporting units is woven by one braided wire, and each of the protrusion branch bodies is formed by another braided wire.

64. The heart valve stent according to claim 63, wherein the braided wire comprises a variable-diameter part, wherein a diameter of the variable-diameter part is larger than that of other parts, and the variable-diameter part is arranged corresponding to the riveted structure.

65. The heart valve stent according to claim 64, wherein the braided wire is made of a variable-diameter memory alloy wire to form the variable-diameter part; or
a memory alloy tube is embedded at an outer periphery of the braided wire, wherein a part having the memory alloy tube is the variable-diameter part.

66. The heart valve stent according to any one of claims 53-65, wherein a connecting ring is formed in a downstream direction of each of the supporting units.

67. A heart valve prosthesis, **characterized by** comprising the heart valve stent according to any one of claims 51-66, and comprising:
a valve leaflet, arranged in the channel and connected to the first supporting bodies of the heart valve stent; and
a first skirt, connecting and covering a space formed between the two first supporting bodies and the third supporting body of the supporting units of the heart valve stent.

68. The heart valve prosthesis according to claim 67, wherein the heart valve prosthesis further comprises a second skirt, wherein the second skirt is arranged around an outer peripheral side of the heart valve stent; an upstream end of the second skirt is connected to the first skirt; and a circumference of the second skirt from the upstream end to a downstream end gradually becomes larger and then gradually becomes smaller.

69. The heart valve prosthesis according to claim 68, wherein an annular first flange is formed on the downstream end of the second skirt, and the first flange faces downstream the heart valve stent.

70. The heart valve prosthesis according to claim 69, wherein an accommodation notch is formed on the first flange and the second skirt, and the accommodation notch is configured to accommodate the supporting body.

71. The heart valve prosthesis according to claim 68, wherein an annular second flange is formed at a peripheral side of the second skirt, and the second flange faces downstream the heart valve stent.

72. The heart valve prosthesis according to claim 71, wherein the second flange is arranged on a peripheral side of a largest circumference of the second skirt.
